# EUROPEAN PATENT APPLICATION

(11) **EP 4 180 446 A1**
(43) Date of publication of application: **17.05.2023**
(21) Application number: 21837916.2
(22) Date of filing: 09.07.2021
(51) Int. Cl.: C07K 7/50, A61K 38/08, A61K 38/10, A61K 38/12, A61K 38/16, A61P 25/00, A61P 25/18, A61P 25/28, A61P 43/00

(54) **CYCLIC PEPTIDE, PEPTIDE COMPLEX, AND DRUG COMPOSITION CONTAINING SAID CYCLIC PEPTIDE AND/OR SAID PEPTIDE COMPLEX**

(30) Priority: 10.07.2020 JP 2020119488
(71) Applicant: The University of Tokyo, Bunkyo-ku, Tokyo 113-8654 (JP)
(72) Inventor: SUGA, Hiroaki, Tokyo 113-8654 (JP); RUMIT, Maini, Tokyo 113-8654 (JP)
(74) Representative: D Young & Co LLP
(86) International application number: PCT/JP2021/026039
(87) International publication number: WO 2022/009992

(57) **Abstract**

The present invention relates to a cyclic peptide having a structure represented by the following formula (1):

-Xaa¹-(Xaa²)ₙ₁-Xaa³-Xaa⁴- (1)

(in the formula (1), Xaa¹ is a basic amino acid, Xaa² is an amino acid, Xaa³ and Xaa⁴ are each independently a basic amino acid or an aromatic amino acid, and n1 is an integer of 0 to 10), or a pharmacologically acceptable salt of the cyclic peptide.

## Description

### Technical Field

The present invention relates to a cyclic peptide, a peptide complex, a pharmaceutical composition containing the cyclic peptide and/or the peptide complex, and the like.

### Background Art

Neurotrophins are endogenous polypeptides that regulate the development, function, and survival of neuronal cells and networks across the whole brain area. Among the neurotrophin family members, a brain-derived neurotrophic factor (BDNF) has recently emerged as a main candidate in potential treatments for neurological and psychiatric disorders (Non-Patent Documents 1 and 2).

It is known that BDNF acts as a natural ligand to a tropomyosin-related receptor kinase B (TrkB) and a neurotrophin receptor p75, which are two receptors of distinct classes, and it binds to TrkB with affinity at least 10 times higher than that of p75 (Non-Patent Document 3). TrkB and its ligand BDNF are widely expressed in the adult mammal brain including the cerebral cortex, hippocampus, brainstem, and spinal cord nuclei (Non-Patent Document 4).

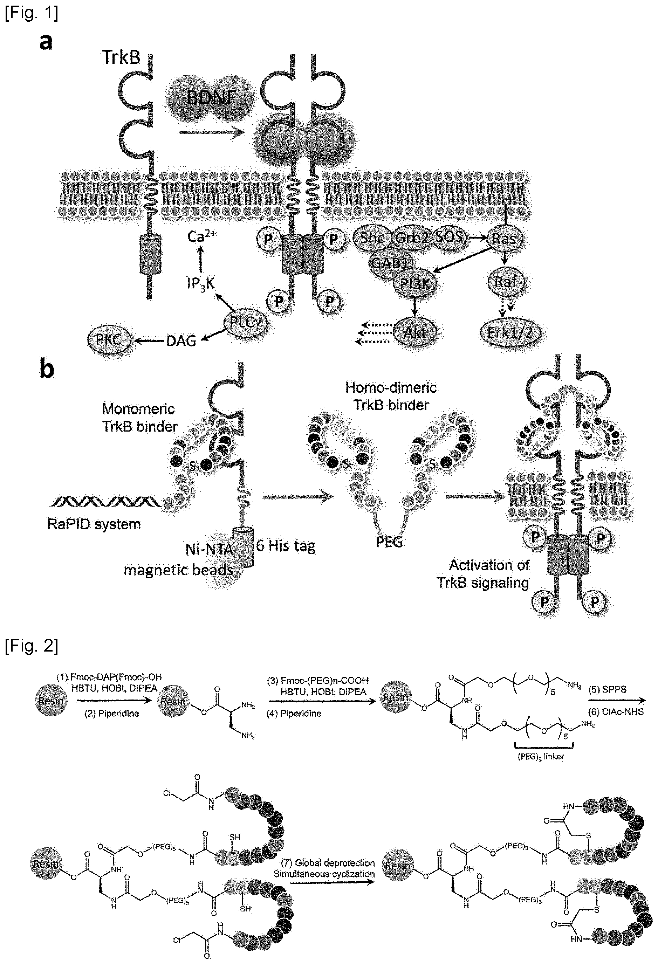

TrkB is a transmembrane tyrosine kinase receptor protein and belongs to the tropomyosin-related kinase (TrK) family. When it binds to BDNF, the tyrosine residue in the vicinity of the cytoplasmic C-terminal domain undergoes autophosphorylation (Fig. 1(a)) (Non-Patent Documents 1 and 5). Phosphorylation of TrkB by BDNF activates three major downstream pathways, that is, PLCγ, PI3K, and MEK-ERK pathways (Non-Patent Document 1).

Phosphorylation of phospholipase C-γ1 (PLCγ1) leads to production of inositol triphosphate (InsP₃) and diacylglycerol (DAG). This production releases the intracellular calcium stores and thereby has an influence on the synaptic plasticity via activation of calcium-dependent protein kinases. Activation of phosphoinositide 3-kinase (PI3K) stimulates protein kinase B (AKT) and in turn, suppresses a pro-apoptotic protein, and finally promotes cell survival (Non-Patent Document 6). Downstream stimulation of mitogen-activated kinase kinase 1/2 (MEK 1/2) and extracellular signal-regulated kinase 1/2 (ERK 1/2) is associated with the neurite elongation, cell division, and neuron survival in which BDNF is involved, in the developmental stage. Further, activation of MEK-ERK pathways is related to maintenance of the neuron structure and function in adult brains (Non-Patent Documents 1 and 6).

In this context, it has been demonstrated that the neurotrophic actions of BDNF have a positive influence on various neurological diseases such as amyotrophic lateral sclerosis (ALS), Parkinson's diseases and Alzheimer's diseases (Non-Patent Documents 7 to 11).

In rodent ALS models, administration of BDNF led to survival of degenerating motor neurons and rescued axotomized motor neurons (Non-Patent Document 7). In non-human primates, infusion of BDNF protein had useful anatomical and behavioral effects and in rodents, the treatment with BDNF prevented the loss of dopaminergic neurons of the substantia nigra. In both of the models, Parkinson's syndrome was a chemically induced one (Non-Patent Documents 9 and 10). In transgenic mice models who expressed a mutation type amyloid precursor protein, BDNF improved learning and memory, and in adult rats after performant path lesion, it suppressed neuronal death (Non-Patent Document 11).

As described above, BDNF therapies have great potential as a treatment method of various neurological diseases, so that a variety of researches on BDNF therapies including a clinical test using recombinant BDNF have been carried out (Non-Patent Documents 12, 13, and 14).

### Citation List

### Non-Patent Documents

Non-Patent Document 1: Liu, C., Chan, C.B., and Ye, K. (2016). 7,8-dihydroxyflavone, a small molecular TrkB agonist, is useful for treating various BDNF-implicated human disorders. Transl Neurodegener 5, 2.
Non-Patent Document 2: Nagahara, A.H., and Tuszynski, M.H. (2011). Potential therapeutic uses of BDNF in neurological and psychiatric disorders. Nat Rev Drug Discov 10, 209-219.
Non-Patent Document 3: Pattarawarapan, M., and Burgess, K. (2003). Molecular basis of neurotrophin-receptor interactions. J Med Chem 46, 5277-5291.
Non-Patent Document 4: Yan, Q., Radeke, M.J., Matheson, C.R., Talvenheimo, J., Welcher, A.A., and Feinstein, S.C. (1997). Immunocytochemical localization of TrkB in the central nervous system of the adult (vol 378, pg 135, 1997). J Comp Neurol 382, 546-547.
Non-Patent Document 5: Klein, R., Nanduri, V., Jing, S.A., Lamballe, F., Tapley, P., Bryant, S., Cordon-Cardo, C., Jones, K.R., Reichardt, L.F., and Barbacid, M. (1991). The trkB tyrosine protein kinase is a receptor for brain-derived neurotrophic factor and neurotrophin-3. Cell 66, 395-403.
Non-Patent Document 6: Yoshii, A., and Constantine-Paton, M. (2010). Postsynaptic BDNF-TrkB signaling in synapse maturation, plasticity, and disease. Dev Neurobiol 70, 304-322.
Non-Patent Document 7: Ekestern, E. (2004). Neurotrophic factors and amyotrophic lateral sclerosis. Neurodegener Dis 1, 88-100.
Non-Patent Document 8: Levivier, M., Przedborski, S., Bencsics, C., and Kang, U.J. (1995). Intrastriatal implantation of fibroblasts genetically engineered to produce brain-derived neurotrophic factor prevents degeneration of dopaminergic neurons in a rat model of Parkinson's disease. J Neurosci 15, 7810-7820.
Non-Patent Document 9: Levivier, M., Przedborski, S., Bencsics, C., and Kang, U.J. (1995). Intrastriatal implantation of fibroblasts genetically engineered to produce brain-derived neurotrophic factor prevents degeneration of dopaminergic neurons in a rat model of Parkinson's disease. J Neurosci 15, 7810-7820.
Non-Patent Document 10: Tsukahara, T, Takeda, M., Shimohama, S., Ohara, O., and Hashimoto, N. (1995). Effects of brain-derived neurotrophic factor on 1-methyl-4-phenyl-1,2,3,6-tetrahydropyridine-induced parkinsonism in monkeys. Neurosurgery 37, 733-739; discussion 739-741.
Non-Patent Document 11: Nagahara, A.H., Merrill, D.A., Coppola, G., Tsukada, S., Schroeder, B.E., Shaked, G.M., Wang, L., Blesch, A., Kim, A., Conner, J.M., et al.
(2009). Neuroprotective effects of brain-derived neurotrophic factor in rodent and primate models of Alzheimer's disease. Nat Med 15, 331-337.
Non-Patent Document 12: Ochs, G., Penn, R.D., York, M., Giess, R., Beck, M., Tonn, J., Haigh, J., Malta, E., Traub, M., Sendtner, M., et al. (2000). A phase I/II trial of recombinant methionyl human brain derived neurotrophic factor administered by intrathecal infusion to patients with amyotrophic lateral sclerosis. Amyotroph Lateral Scler Other Motor Neuron Disord 1, 201-206.
Non-Patent Document 13: Thoenen, H., and Sendtner, M. (2002). Neurotrophins: from enthusiastic expectations through sobering experiences to rational therapeutic approaches. Nat Neurosci 5 Suppl, 1046-1050.
Non-Patent Document 14: Chao, M.V. (1994). The p75 neurotrophin receptor. J Neurobiol 25, 1373-1385.

### Summary

### Technical Problem

However, conventional BDNF therapies have not been established as a therapeutic method having a sufficient effect because of insufficient delivery of BDNF and a short in vivo half-life (Non-Patent Documents 12 and 13). BDNF is also known to activate cell death pathway when it binds to p75 (Non-Patent Document 14) and its dosage should be adjusted to mitigate an off-target effect.

There is therefore a demand for a novel compound capable of replacing BDNF and binding to TrkB.

The present invention has been made in consideration of the aforesaid problems and the purpose is to provide a novel compound capable of binding to TrkB and/or a compound capable of selectively inducing downstream TrkB signaling.

### Solution to Problem

The present inventors have made intensive studies to achieve the aforesaid purpose. As a result, it has been found that a cyclic peptide having a particular structure binds to TrkB and a peptide complex of the cyclic peptide having a particular structure selectively induces downstream TrkB signaling, leading to the completion of the present invention.

The present invention is as follows.
[1] A cyclic peptide having a structure represented by the following formula (1):

   -Xaa¹-(Xaa²)ₙ₁-Xaa³-Xaa⁴- (1)

   (in the formula (1),
   Xaa¹ is a basic amino acid,
   Xaa² is an amino acid,
   Xaa³ and Xaa⁴ are each independently a basic amino acid or an aromatic amino acid, and
   n1 is an integer of 0 to 10), or a pharmacologically acceptable salt of the cyclic peptide.
[2] The cyclic peptide or pharmacologically acceptable salt thereof as described in [1], wherein the structure represented by the formula (1) is any of the structures represented by the following formula (2):

   -Arg-(Xaa²)ₙ₁-Phe-Trp- (2)

   (in the formula (2),
   Xaa² and n1 have the same meanings as described above).
[3] The cyclic peptide or pharmacologically acceptable salt thereof as described in [1] or [2], wherein the cyclic structure of the cyclic peptide contains an N-CO-CH₂-S structure.
[4] The cyclic peptide or pharmacologically acceptable salt thereof as described in [3], wherein the N is derived from the amino group of tyrosine.
[5] The cyclic peptide or pharmacologically acceptable salt thereof as described in [3] or [4], wherein the S is derived from the thiol group of cysteine.
[6] The cyclic peptide or pharmacologically acceptable salt thereof as described in any one of [1] to [5], wherein the number of amino acid residues of the cyclic structure of the cyclic peptide is 6 to 20.
[7] A cyclic peptide containing any one of the cyclic structures represented by the following formulas (3) to (8): 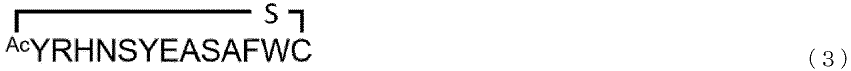 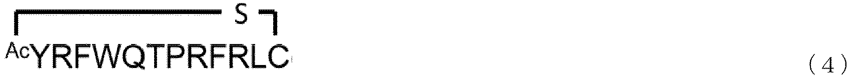 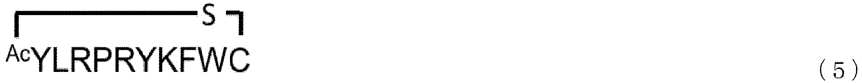 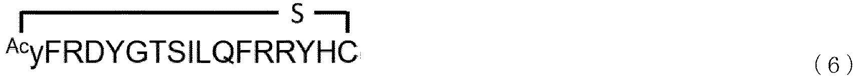 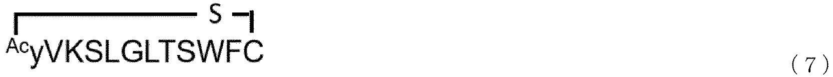 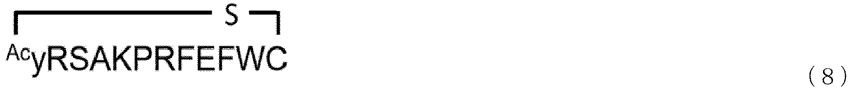 (in the formulas (3) to (8), Ac is -CH₂-CO-, Y is L-tyrosine, and y is D-tyrosine), or a pharmacologically acceptable salt of the cyclic peptide.
[8] The cyclic peptide or pharmacologically acceptable salt thereof as described in any one of [1] to [7], wherein the cyclic peptide further contains a linear structure having 1 to 20 amino acids.
[9] A peptide complex containing two cyclic peptides as described in any one of [1] to [7], one of the cyclic peptides being linked to the other cyclic peptide via a linker, or a pharmacologically acceptable salt of the peptide complex.
[10] The peptide complex or pharmacologically acceptable salt thereof as described in [9], wherein the two cyclic peptides are identical.
[11] The peptide complex or pharmacologically acceptable salt thereof as described in [9] or [10], wherein the linker has a length of 20 Å or more and 200 Å or less.
[12] The peptide complex or pharmacologically acceptable salt thereof as described in any one of [9] to [11], inducing phosphorylation of TrkB.
[13] A pharmaceutical composition containing at least one selected from the group consisting of the cyclic peptide as described in any one of [1] to [7] and pharmacologically acceptable salt thereof and the peptide complex as described in any one of [9] to [12] and pharmacologically acceptable salt thereof.
[14] The pharmaceutical composition as described in [13] to be used for the treatment or prevention of neurodegenerative diseases, neurodevelopmental disorders, or neuropsychiatric diseases.

The present invention also provides the following further embodiments.

A pharmaceutical composition containing at least one selected from the group consisting of the cyclic peptide as described in any one of [1] to [7] and pharmacologically acceptable salt thereof and the peptide complex as described in any one of [9] to [12] and pharmacologically acceptable salt thereof; and a pharmacologically acceptable additive.

The pharmaceutical composition as described in [15] to be used for the treatment or prevention of neurodegenerative diseases, neurodevelopmental disorders, or neuropsychiatric diseases.

A TrkB binder containing at least one selected from the group consisting of the cyclic peptide as described in any one of [1] to [7] and pharmacologically acceptable salt thereof and the peptide complex as described in any one of [9] to [12] and pharmacologically acceptable salt thereof.

The TrkB binder as described in [16], wherein the TrkB binder is a human TrkB binder.

The TrkB binder as described in [16] or [16A], inducing phosphorylation of TrkB.

An inducer of TrkB phosphorylation, an inducer of neuronal gene activation, or a promoter of axon outgrowth, containing at least one selected from the group consisting of the cyclic peptide as described in any one of [1] to [7] and pharmacologically acceptable salt thereof and the peptide complex as described in any one of [9] to [12] and pharmacologically acceptable salt thereof.

A method of treating or preventing neurodegenerative diseases, neurodevelopmental disorders, or neuropsychiatric diseases, including administering at least one selected from the group consisting of the cyclic peptide as described in any one of [1] to [7] and pharmacologically acceptable salt thereof and the peptide complex as described in any one of [9] to [12] and pharmacologically acceptable salt thereof.

Use of at least one selected from the group consisting of the cyclic peptide as described in any one of [1] to [7] and pharmacologically acceptable salt thereof and the peptide complex as described in any one of [9] to [12] and pharmacologically acceptable salt thereof for the manufacture of a pharmaceutical composition for treating or preventing neurodegenerative diseases, neurodevelopmental disorders, or neuropsychiatric diseases.

At least one selected from the group consisting of the cyclic peptide as described in any one of [1] to [7] and pharmacologically acceptable salt thereof and the peptide complex as described in any one of [9] to [12] and pharmacologically acceptable salt thereof for the treatment or prevention of neurodegenerative diseases, neurodevelopmental disorders, or neuropsychiatric diseases.

The cyclic peptide or pharmacologically acceptable salt thereof as described in any one of [1] to [7] or the peptide complex or pharmacologically acceptable salt thereof as described in any one of [9] to [12] for the treatment or prevention of neurodegenerative diseases, neurodevelopmental disorders, or neuropsychiatric diseases.

Use of at least one selected from the group consisting of the cyclic peptide as described in any one of [1] to [7] and pharmacologically acceptable salt thereof and the peptide complex as described in any one of [9] to [12] and pharmacologically acceptable salt thereof for the treatment or prevention of neurodegenerative diseases, neurodevelopmental disorders, or neuropsychiatric diseases.

The present invention may also be the following still further embodiments.

The TrkB binder, the inducer of TrkB phosphorylation, the inducer of neuronal gene activation, or the promoter of axon outgrowth in [16] to [16C] may be used for the treatment or prevention of neurodegenerative diseases, neurodevelopmental disorders, or neuropsychiatric diseases.

At least one selected from the group consisting of the cyclic peptide as described in any one of [1] to [7] and pharmacologically acceptable salt thereof and the peptide complex as described in any one of [9] to [12] and pharmacologically acceptable salt thereof may be used for the treatment or prevention of neurodegenerative diseases, neurodevelopmental disorders, or neuropsychiatric diseases as described in [17] to [20] by binding to TrkB (preferably, binding to human TrkB), induction of TrkB phosphorylation, induction of neuronal gene activation, or promotion of axon outgrowth.

The cyclic peptide as described in any one of [1] to [7] and pharmacologically acceptable salt thereof and the peptide as described in any one of [9] to [12] complex and pharmacologically acceptable salt thereof may be any combination of aspects described herein and preferred, more preferred, and still more preferred aspects (all these aspects may be called "preferred aspects", collectively) described herein.

### Advantageous Effects of Invention

The present invention provides a novel compound capable of binding to TrkB and/or a compound capable of selectively inducing the downstream TrkB signaling.

### Brief Description of Drawings

Fig. 1(a) shows that BDNF induces phosphorylation of TrkB, resulting in activation of three main downstream pathways, that is, phospholipase Cγ (PLCγ), phosphoinositide-3-kinase (PI3K), and mitogen-activated kinase kinase-extracellular signal-regulated kinase (MEK-ERK) pathways. In the present specification, it has been revealed that the MEK-ERK pathway to which main attention has been paid is involved in the outgrowth of a neurite in the development stage, cell differentiation, and survival of nerve cells. Fig. 1(b) shows conversion of an anti-TrkB macrocyclic peptide discovered by the RaPID system into a macrocyclic homodimer with a polyethylene glycol (PEG) linker having an adequate length. The homodimeric peptide thus obtained was used further for the discovery of a BDNF mimic.
Fig. 2 shows a method of synthesizing a homodimeric macrocyclic peptide using solid-phase peptide synthetic chemistry.

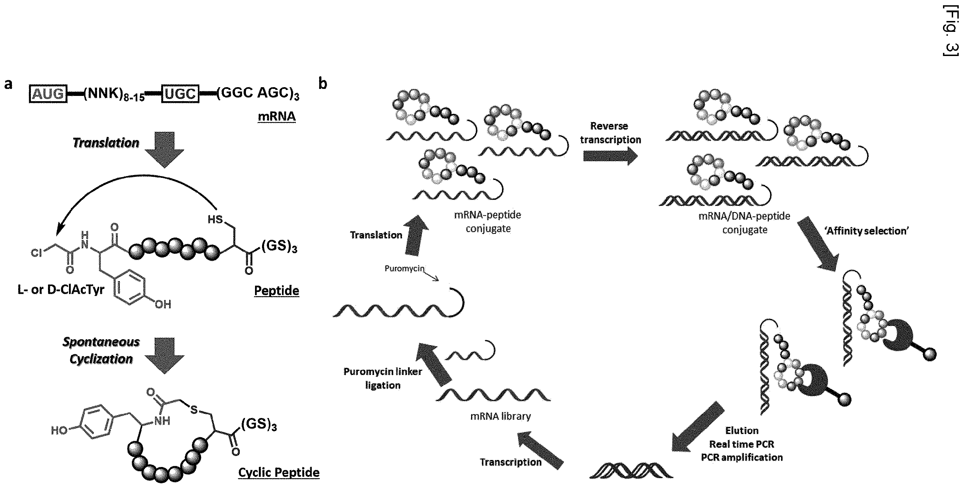

Fig. 3(a) shows a formation strategy of a thioether-cyclic peptide library using the flexible in vitro translation (FIT) system. Fig. 3(b) is a schematic diagram of RaPID screening approach used herein.

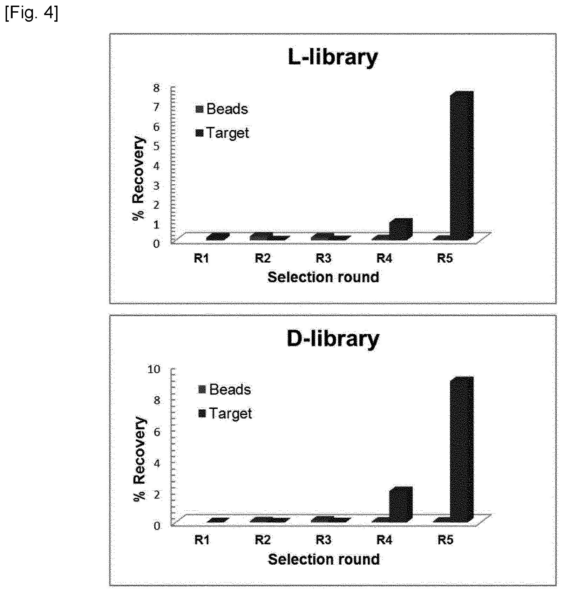

Fig. 4 shows the selection results of peptide sequences identified by the first RaPID screening for the affinity for TrkB. The progress of the affinity selection of each of the D-library and the L-library is indicated as a percent of the number of molecules that bind to a TrkB beads complex (red) or simple beads (blue) to the total number of molecules yielded by the affinity selection in each round.

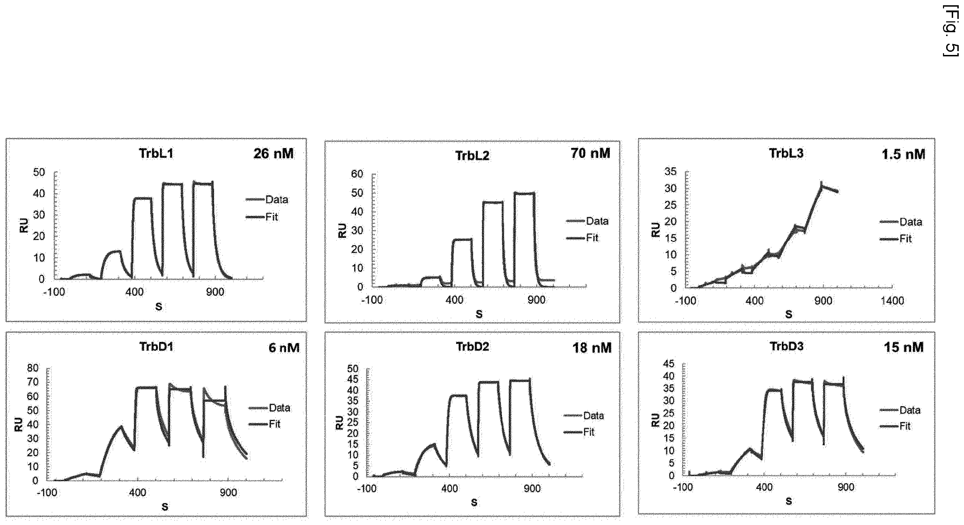

Fig. 5 shows SPR sensorgrams of monomeric peptides that bind to TrkB analyzed by Biacore T200 within a concentration range of 1 to 1000 nM. Data line and fit line are shown in blue and red, respectively.

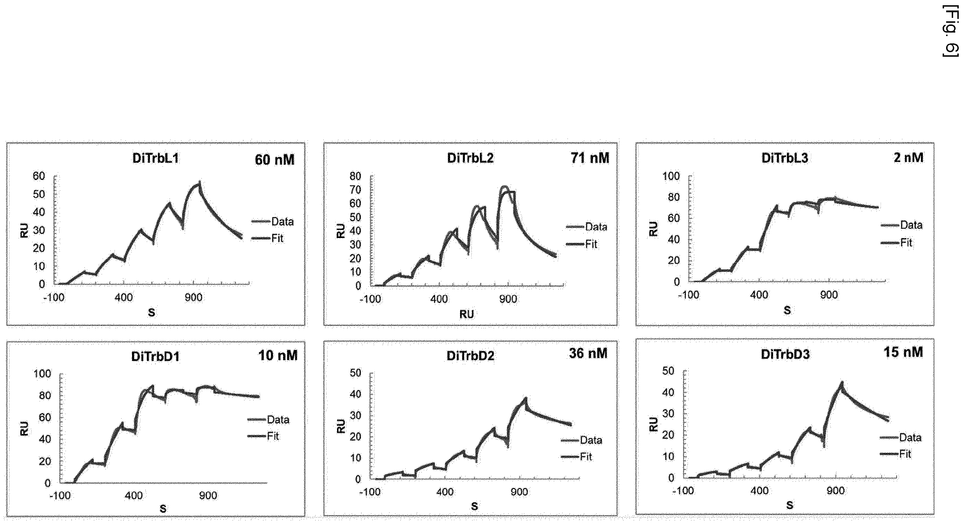

Fig. 6 shows SPR sensorgrams of homodimeric peptides that bind to TrkB analyzed by Biacore T200 within a concentration range of 1 to 1000 nM. Data line and fit line are shown in blue and red, respectively.

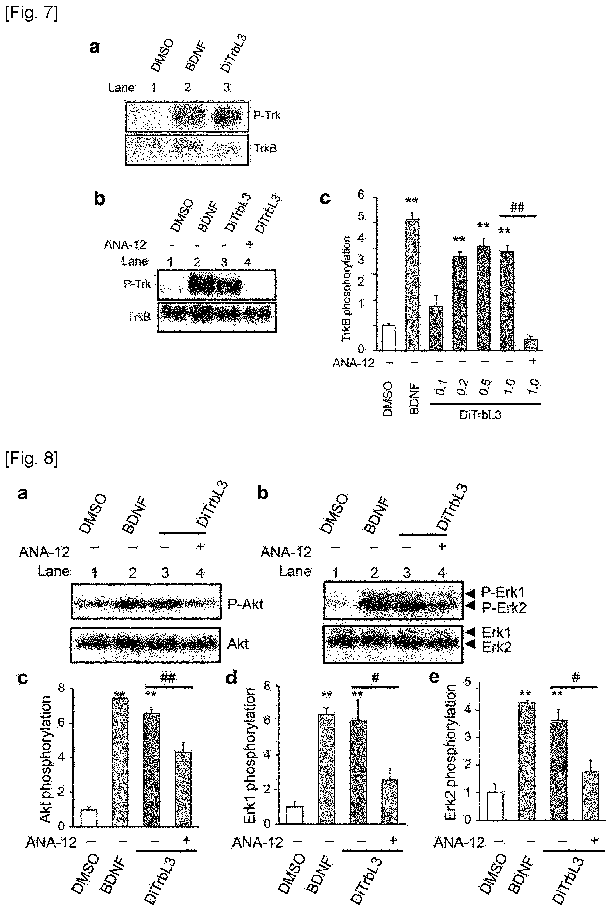

Fig. 7(a) shows the typical screening results of DiTrbL3 by the western blot of phosphorylated Trk (P-Trk). The primary mouse hippocampal neurons (DIV6) were treated with DMSO, BDNF (0.1 nM), or DiTrbL3 (1 nM) for 15 minutes. For the internal control (TrkB), an anti-TrkB antibody was used. Fig. 7(b) shows the evaluation of target specificity of DiTrbL3 by using ANA-12, a selective inhibitor to the TrkB kinase domain. Thirty minutes before the peptide treatment, ANA-12 (100 µM) was added to a medium. Fig. 7(c) shows the semiquantitative results of the Trk phosphorylation. The TrkB phosphorylation level was normalized with total TrkB, followed by normalization with an average of the DMSO treatment group. The error bar represents the standard error of mean (s.e.m.) and in each group, n=3. **: p<0.01 (comparison with DMSO), ##: p<0.01 (comparison between 100 µM ANA-12 and 1 nM DiTrbL3, and 1 nM DiTrbL3). For statistical analysis, one-way ANOVA was used, followed by a Tukey-Kramer multiple comparison test.
Fig. 8 shows the treatment of primary mouse hippocampal neurons (DIV6) for 15 minutes with DMSO (negative control of peptide), BDNF (0.1 nM), or DiTrbL3 (1 nM). Thirty minutes before the peptide treatment, ANA12 (100 µM) was added to a medium. Fig. 8(a) shows the typical western blot of phosphorylated Akt (P-Akt in S473). Fig. 8(b) shows the typical western blot of phosphorylated Erk1/2 (P-Erk1/2). Figs. 8(c) to (e) show the quantitative results of the phosphorylation level of (c) Akt, (d) Erk1, and (e) Erk2. The arrows show Erk1 (44 kDa) and Erk2 (42 kDa), respectively. The relative phosphorylation level of each kinase was normalized to the mean of the DMSO treatment group. The error bar represents the standard error (s.e.m.) and in each group, n=3. **: p<0.01 (comparison with DMSO), ##: p<0.01, #: p<0.05 (comparison between ANA-12 and 1 nM DiTrbL3, and 1 nM DiTrbL3). For statistical analysis, one-way ANOVA was used, followed by a Tukey-Kramer multiple comparison test.

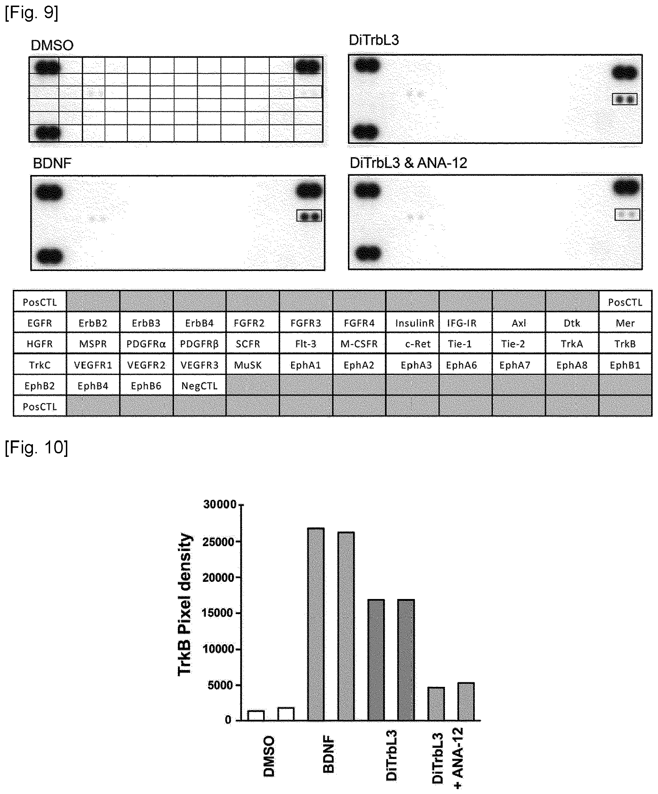

Fig. 9 shows the results of a phospho-RTK array. A commercially available phospho-RTK array including 40 kinds of RTK were used (bottom). The primary hippocampal neurons (DIV6) were incubated for 15 minutes with a stimulant. Thirty minutes before the DiTrbL3 treatment, ANA-12 (100 µM) was added. Each spot shows the phosphorylation level of each RTK. The red square portion shows overlapped TrkB spots.
Fig. 10 shows the densitometric analysis of TrkB in the RTK array. The phosphorylation level of each spot of TrkB was normalized to the mean of the DMSO treatment group.

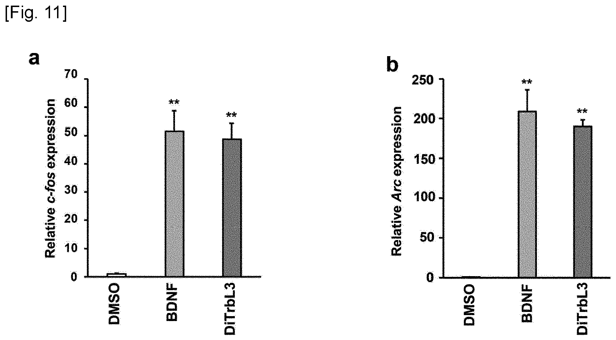

Fig. 11 shows evaluations of the mRNA expression of (a) c-fos and (b) Arc by qRT-PCR after 1-hr treatment of cultured cortical neurons (DIV6) with BDNF (1 nM) or DiTrbL3 (1 nM). In each treatment, n=3. The error bar represents the standard error of mean (s.e.m.). **: p<0.01 (comparison with DMSO). For statistical analysis, one-way ANOVA was used, followed by a Tukey-Kramer multiple comparison test.

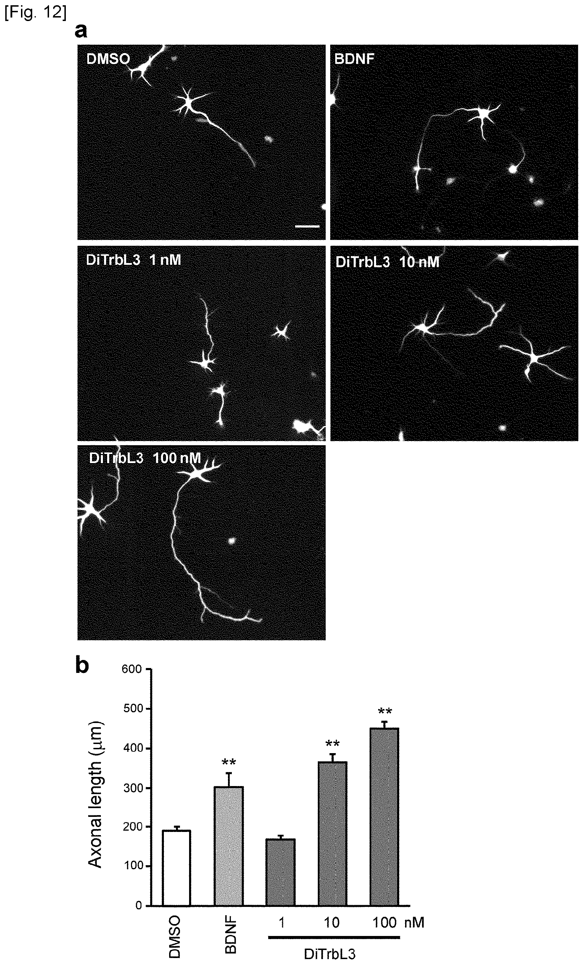

Fig. 12(a) is the typical images of primary hippocampal neurons treated with DiTrbL3 peptide. The hippocampal neurons were treated to DIVa to 3 with 1, 10, or 100 nM DiTrbL3, or 2 nM BDNF and they were subjected to nuclear staining (DAPI; blue) and in addition, immunocytochemistry with an anti-Tau antibody (red) and an anti-MAP2 antibody (green). The axon was identified as a Tau positive and MAP2 negative neurite. The length of each axon was quantified by Neuron J44. The scale bar is 50 µm. Fig. 12(b) shows the quantitative results of an axon outgrowth experiment (n=60 in DMSO, n=20 in BDNF, n=30 in each concentration of DiTrbL3). The error bar represents the standard error of the mean (s.e.m.). **: p<0.01 (comparison with DMSO). For statistical analysis, one-way ANOVA was used, followed by a Tukey-Kramer multiple comparison test.

### Description of Embodiments

The embodiments of the present invention will hereinafter be described in detail. The present invention is not limited to or by the following embodiments and can be performed in various modified forms within the scope of the gist of the invention.

The term "pharmacologically acceptable salt" as used herein embraces, for example, salts of a base or an acid which are pharmaceutically acceptable.

Nonlimiting specific examples of the pharmacologically acceptable salt include addition salts of an inorganic acid (hydrochloric acid, hydrobromic acid, hydroiodic acid, sulfuric acid, phosphoric acid, or the like), addition salts of an organic acid (p-toluenesulfonic acid, methanesulfonic acid, oxalic acid, p-bromophenylsulfonic acid, carboxylic acid, succinic acid, citric acid, benzoic acid, acetic acid, or the like), addition salts of an inorganic base (ammonium hydroxide, alkali or alkaline earth metal hydroxide, carbonate, bicarbonate, or the like), and addition salts of an amino acid.

### [Cyclic peptide]

The cyclic peptide and pharmacologically acceptable salt thereof according to the present invention have, as a partial structure of a cyclic structure, a structure represented by the following formula (1):

-Xaa¹-(Xaa²)ₙ₁-Xaa³-Xaa⁴- (1)

In the formula (1),
Xaa¹ is a basic amino acid,
Xaa² is an amino acid,
Xaa³ and Xaa⁴ are each independently a basic amino acid or an aromatic amino acid, and
n1 is an integer of 0 to 10.

The term "cyclic peptide" as used herein means a cyclic peptide at least having, in the molecule thereof, a cyclic structure composed of 5 or more amino acids. The molecular structure of the cyclic peptide may have, in addition to the cyclic structure, a chain structure in which amino acids are linked via peptide bonding or may have a structure other than the peptide structure.

The term "cyclic structure" as used herein means a closed-ring structure formed in a molecule by binding of two amino acids of a linear peptide, which are separated by three or more amino acid residues, to each other directly or via a linker or the like.

The term "separated by three or more amino acid residues" as used herein means that the number of the amino acid residues present between the two amino acids forming the closed ring structure is at least 3.

The closed ring structure in the cyclic structure is not particularly limited, and it is formed by the covalent bonding of two amino acids optionally via a linker or the like.

Examples of the covalent bonding between two amino acids include disulfide bonding, peptide bonding, alkyl bonding, alkenyl bonding, ester bonding, thioester bonding, ether bonding, thioether bonding, phosphonate ether bonding, azo bonding, C-S-C bonding, C-N-C bonding, C=N-C bonding, amide bonding, lactam bridging, carbamoyl bonding, urea bonding, thiourea bonding, amine bonding, and thioamide bonding.

The covalent bonding between two amino acids may be formed by bonding between the respective side chains of the two amino acids, bonding between the respective main chains of the two amino acids, bonding between the side chain and the main chain of the two amino acids, or the like. When the bonding of two amino acids is between the main chains of the amino acids, a closed ring structure is formed by peptide bonding.

The cyclic structure is not limited to that formed by bonding between the N-terminal and C-terminal amino acids of a linear peptide, but it may be formed by bonding between a terminal amino acid and a non-terminal amino acid or bonding between non-terminal amino acids. When one of the amino acids that binds to form a cyclic structure is a terminal amino acid and the other one is a non-terminal amino acid, the resulting cyclic peptide has a cyclic structure having a straight-chain peptide attached thereto like a tail.

When the straight-chain peptide attaches like a tail to the cyclic structure, the straight-chain peptide preferably binds to the C terminal of the amino acids which form part of the cyclic structure of the linear peptide. In other words, the cyclic structure is preferably formed by bonding between the N-terminal amino acid of the linear peptide and an amino acid other than that at the C terminal.

Examples of the amino acid of the cyclic structure include natural amino acids and artificial amino acid variants and/or derivatives thereof, for example, natural proteinogenic L-amino acids, unnatural amino acids, and chemically synthesized compounds having properties known in the art as characteristics of an amino acid.

The proteinogenic amino acids are, when expressed by a three-letter code known in the art, Arg, His, Lys, Asp, Glu, Ser, Thr, Asn, Gln, Cys, Gly, Pro, Ala, Ile, Leu, Met, Phe, Trp, Tyr, and Val. The proteinogenic amino acids are, when expressed by a one-letter code known in the art, R, H, K, D, E, S, T, N, Q, C, G, P, A, I, L, M, F, W, Y, and V.

The term "non-proteinogenic amino acids" means natural or unnatural amino acids other than the proteinogenic amino acids.

Examples of the unnatural amino acids include amino acids having a main chain structure different from that of natural amino acids (α,α-disubstituted amino acids (such as α-methylalanine), N-alkylamino acids, D-amino acids, β-amino acids, γ-amino acids, δ-amino acids, α-hydroxy acids, and the like); amino acids having a side-chain structure different from that of natural amino acids (such as norleucine and homohistidine); amino acids having extra methylene in the side chain thereof (such as "homo"amino acids, homophenylalanine, and homohistidine); and amino acids in which a carboxylic acid functional group in the side chain has been substituted by a sulfonic acid group (such as cysteic acid). Specific examples of the unnatural amino acid include amino acids described in WO2015/030014.

The number of the amino acid residues of the cyclic structure is not particularly limited as long as it is 5 or more. The number may be, for example, 6 or more, 7 or more, 8 or more, 9 or more, or 10 or more and 30 or less, 25 or less, 20 or less, 17 or less, or 15 or less.

The number of amino acids of the cyclic structure is usually 5 or more and 30 or less. Within a range of 5 or more and 30 or less, the number of amino acids of the cyclic structure may be set at 6 or more, 8 or more, or 10 or more and 30 or less, 25 or less, 20 or less, or 15 or less.

The number of amino acids of the cyclic structure may be set at 6 or more and 20 or less, 8 or more and 20 or less, 8 or more and 19 or less, 9 or more and 18 or less, 9 or more and 17 or less, 10 or more and 17 or less, or 10 or more and 16 or less.

The number of amino acids of the cyclic structure is preferably 8 or more and 17 or less, more preferably 9 or more and 15 or less, still more preferably 10 or more and 14 or less from the standpoint of having enhanced affinity for TrkB.

In the present invention, the cyclic peptide may be a modified one such as phosphorylated, methylated, acetylated, adenylated, ADP-ribosylated, glycosylated, or polyethylene glycol-added one or it may be a fused one with another peptide and/or protein. Further, the cyclic peptide may be biotinated via an appropriate linker.

In the present invention, the cyclic peptide may also be a dimer having, in the molecule thereof, two cyclic structures and obtained by binding two cyclic peptides, each having one cyclic structure, to each other via a linker structure, or it may have an intramolecular lactam bridge structure obtained by intramolecularly forming a lactam structure. In the present specification, a cyclic peptide dimer in which two cyclic peptides having a cyclic structure having a structure represented by the aforesaid formula (1) have bound to each other via a linker structure will be called "peptide complex" as will be descried later.

The linker structure that links two cyclic peptides is not particularly limited and a linker having a structure known in the peptide synthesis field as a linker that links two peptides to each other can be used.

The intramolecular lactam bridge structure may be formed by binding the respective side chains of amino acids of the cyclic peptide. For example, an intramolecular lactam structure is formed by binding the side-chain amino group of Lys to the side-chain carboxyl group of Asp or Glu to form a peptide bond and thereby form an intramolecular lactam structure. Such a cyclic peptide has, in the molecule thereof, another cyclic structure as a bridge structure. Instead of Lys, for example, DAP, DAB, or Orn may bind to Asp or Glu.

The basic amino acid in the present specification is not particularly limited as long as it is an amino acid having, in the molecule thereof, a side chain showing basicity and examples of it as a proteinogenic amino acid include Arg, Lys, His, and Trp.

The aromatic amino acid in the present specification is not particularly limited as long as it is an amino acid having, in the molecule thereof, an aromatic ring in the side chain and examples of it as a proteinogenic amino acid include Trp, His, Phe, and Tyr.

In the structure represented by the formula (1), Xaa¹ is a basic amino acid and may be Arg, Lys, His, or Trp, preferably Arg or Lys, more preferably Arg.

In the structure represented by the formula (1), Xaa³ and Xaa⁴ are each independently a basic amino acid or an aromatic amino acid and Xaa³-Xaa⁴ may be any combination of a basic amino acid and a basic amino acid, a basic amino acid and an aromatic amino acid, an aromatic amino acid and a basic amino acid, or an aromatic amino acid and an aromatic amino acid. In the Xaa³-Xaa⁴, the basic amino acid may be Arg, Lys, His, or Trp, preferably Arg or Lys and the aromatic amino acid may be Trp, His, Phe, or Tyr, preferably Trp, Phe, or Tyr, more preferably Trp or Phe. Xaa³-Xaa⁴ may be any combination of Lys or Arg as the basic amino acid and Phe, Trp, or Tyr as the aromatic amino acid. Examples include Phe-Trp, Phe-Arg, Tyr-Arg, Arg-Tyr, Tyr-Trp, Trp-Phe, Arg-Lys, and Arg-Phe. As one preferred aspect of Xaa³-Xaa⁴, for example, Phe-Trp, Arg-Tyr, and Trp-Phe can be given.

In the structure represented by the formula (1), n1 is an integer of 0 to 10 and within this range, it is preferably 1 or more, 2 or more, 3 or more, 4 or more, 5 or more, or 6 or more and is preferably 9 or less or 8 or less.

In the formula (1), Xaa¹, Xaa², Xaa³, Xaa⁴, and n1 may be a structure having any combination of the preferred aspects described as Xaa¹, Xaa², Xaa³, Xaa⁴, and n1.

In the present invention, the structure represented by the formula (1) is preferably a structure represented by the following formula (2):

-Arg-(Xaa²)ₙ₁-Phe-Trp- (2)

In the formula (2), Xaa² and n1 have the same meanings as Xaa² and n1 described in the structure represented by the formula (1) and may be a structure having any combination of the preferred aspects described as Xaa² and n1.

The cyclic peptide of the present invention preferably contains, as a cyclic structure, a structure represented by the following formula (9):

(Z¹)ₘ-Xaa¹-(Xaa²)ₙ₁-Xaa³-Xaa⁴-(Z²)ₙ (9)

In the formula (9), Xaa¹, Xaa², Xaa³, Xaa⁴, and n1 have the same meanings as Xaa¹, Xaa², Xaa³, Xaa⁴, and n1 described in the structure represented by the formula (1) and may be a structure having any combination of preferred aspects described as Xaa¹, Xaa², Xaa³, Xaa⁴, and n1.

The formula (9) is a structure having (Z¹)ₘ bound to the N-terminal side of the structure represented by the formula (1) and (Z²)ₙ bound to the C-terminal side of the structure represented by the formula (1).

In the aforesaid formula (9), m and n are each any integer of 1 or more.

It is preferred that Z¹ and Z² are each independently selected from any amino acid and that one amino acid as Z¹ and one amino acid as Z² form a cyclic structure to form the cyclic peptide. The cyclic structure may be formed by bonding between N-terminal Z¹ and C-terminal Z², may be formed by bonding between N-terminal Z¹ and non-C-terminal Z², may be formed by bonding between non-N-terminal Z¹ and C-terminal Z², or may be formed by bonding between non-N-terminal Z¹ and non-C-terminal Z².

Since the cyclic peptide formed by bonding of either a non-terminal Z¹ or Z² has a structure having a straight chain peptide attached to the cyclic structure like a tail, the cyclic structure is preferably formed by bonding between N-terminal Z¹ and non-C-terminal Z². Alternatively, Z¹ and Z² may form a cyclic structure via a linker.

With respect to m and n, m+n is 2 or more and m and n are preferably independently selected so that m+n falls within a range of 2 or more and 20 or less. With respect to m and n, m and n are preferably independently selected so that m+n falls within a range of 2 or more and 20 or less and the number of amino acids of the cyclic structure falls in the aforesaid preferable range.

Z¹ and Z² may contain, in addition to the amino acid forming the cyclic structure, an amino acid having a branched side chain such as Leu, Val, or Ile, an amino acid having a hydroxyl group on the side chain such as Ser or Thr, a basic amino acid, or an aromatic amino acid, and they preferably contain Leu or Ser.

In the formula (9), Xaa¹, Xaa², Xaa³, Xaa⁴, Z¹, Z², m, n, and n1 may be a structure having any combination of the preferred aspects described as Xaa¹, Xaa², Xaa³, Xaa⁴, Z¹, Z², m, n, and n1.

The partial structure -Xaa¹-(Xaa²)ₙ₁-Xaa³-Xaa⁴- in the formula (9) is preferably a structure represented by the formula (2). In this case, the cyclic peptide of the present invention contains, as a cyclic structure, the structure represented by the following formula (10):

(Z¹)ₘ-Arg-(Xaa²)ₙ₁-Phe-Trp-(Z²)ₙ (10)

In the formula (10), Xaa², Z¹, Z², m, n, and n1 have the same meanings as Xaa², Z¹, Z², m, n, and n1 described in the structures represented by the formula (1) and the formula (9), and it may be a structure having any combination of the preferred aspects described as Xaa², Z¹, Z², m, n, and n1.

The cyclic peptide preferably has an N-CO-CH₂-S structure (that is, -NH-CO-CH₂-S- structure) and the cyclic structure may be formed via the N-CO-CH₂-S structure. The cyclic structure is preferably formed by bonding between an acetyl group which has been bound to the N-terminal amino group of a peptide which has not yet formed the cyclic structure and whose H has been substituted by a leaving group X and a thiol group in the C-terminal side cysteine (Cys). The acetyl group whose H has been substituted by a leaving group X is not particularly limited and examples include a chloroacetyl group. Examples of such an aspect include a cyclic structure formed by bonding between XCH₂CO and a thiol group of Cys in a peptide represented by the following formula (11):

XCH₂CO-(Z³)-(Z⁴)ₚ-Xaa¹-(Xaa²)ₙ₁-Xaa³-Xaa⁴- (Z⁵)_{q}-Cys-(Xaa')ᵣ (11)

In the formula (11), Z³, Z⁴, Z⁵, and Xaa' are each independently any amino acid, p is an integer of m-1, and q is an integer of n-1. They are each an integer of 0 or more and 18 or less. As p and q, they are independently selected as needed so that p+q falls within a range of 0 or more and 18 or less and the number of amino acids of the cyclic structure falls within the aforesaid preferred range.

In the formula (11), Xaa¹, Xaa², Xaa³, Xaa⁴, and n1 have the same meanings as Xaa¹, Xaa², Xaa³, Xaa⁴, and n1 described in the structures represented by the formula (1) and the formula (9), and the structure may have any combination of the preferred aspects described as Xaa¹, Xaa², Xaa³, Xaa⁴, and n1 in the structures represented by the formula (1) and the formula (9). In the formula (11), Z⁴ and Z⁵ may be a structure having any combination of the preferred aspects described as Z¹ and Z² described in the structure represented by the formula (9).

Z³ is preferably Tyr and it may be either a D-form or L-form.

Xaa's are each independently preferably Gly or Ser.

Although r is not particularly limited as long as it is an integer of 0 or more, it may be 20 or less or 10 or less and 1 or more, 3 or more, or 5 or more. r is preferably 0 to 20.

Examples of (Xaa')ᵣ include a structure represented by (-Gly-Ser)ᵣ₁ (wherein r1 is an integer of 0 to 10).

In the present specification, when r is 0, the cyclic peptide does not have Xaa' and when r is an integer of 1 or more, the (Xaa')ᵣ in the cyclic peptide corresponds to a chain structure in which amino acids are linked by peptide bonding.

The cyclic peptide may have a cyclic structure other than that formed by an N-CO-CH₂-S structure. For example, it may be a cyclic peptide obtained by cyclization of an amino acid having Functional group 1 and an amino acid having Functional group 2 corresponding thereto, each shown in the following Table 1. In this aspect, for example, when the structure represented by the aforesaid formula (10) is contained as a cyclic structure, at least one Z¹ may be an amino acid having Functional group 1 and at least one Z² may be an amino acid having Functional group 2 corresponding thereto; or at least one Z¹ may be an amino acid having Functional group 2 and at least one Z² may be an amino acid having Functional group 1 corresponding thereto.

Either one of Functional group 1 and Functional group 2 may be placed on the N-terminal side; they may be placed at the N-terminal and C-terminal, respectively; one of them may be placed as a terminal amino acid and the other one may be placed as a non-terminal amino acid; or both may be placed as a non-terminal amino acid.

A bond formed by Functional group 1 and Functional group 2 may be regarded as a chemical crosslink structure for forming a molecular cyclic structure in a cyclic peptide.

**[Table 1]**

| | Functional group1 | Functional group2 |
|---|---|---|
| (A) | 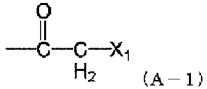 | HS- (A-2) |
| (B) | -C≡C-H (B-1) | N₃- (B-2) |
| (C) | -Ar-CH₂NH₂ (C-1) | 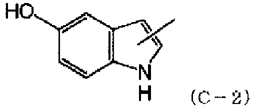 |
| (D) | **-**C≡C-CH₂-X₁ (D-1) | **HS-** (D-2) |
| (R) | -Ar-CH₂₋X₁ (E- 1) | HS- (E-2) |

In the chemical structural formula in Table 1, X₁ is a leaving group and Ar is a substituted or unsubstituted aromatic ring. Examples of the leaving group include halogen atoms such as Cl, Br, and I.

As an amino acid having Functional group (A-1), for example, a chloroacetylated amino acid can be used. Examples of the chloroacetylated amino acid include N-chloroacetyl-L-alanine, N-chloroacetyl-L-phenylalanine, N-chloroacetyl-L-tyrosine, N-chloroacetyl-L-tryptophan, N-3-(2-chloroacetamido)benzoyl-L-phenylalanine, N-3-(2-chloroacetamido)benzoyl-L-tyrosine, N-3-(2-chloroacetamido)benzoyl-L-tryptophan, β-N-chloroacetyl-L-diaminopropanoic acid, γ-N-chloroacetyl-L-diaminobutyric acid, 5-N-chloroacetyl-L-ornithine, and ε-N-chloroacetyl-L-lysine, and D-amino acid derivatives corresponding thereto.

As the amino acid having Functional group (A-1), N-chloroacetyl-L-tyrosine and N-chloroacetyl-D-tyrosine are preferred.

Examples of an amino acid having Functional group (A-2) include cysteine, homocysteine, mercaptonorvaline, mercaptonorleucine, 2-amino-7-mercaptoheptanoic acid, and 2-amino-8-mercaptooctanoic acid.

As the amino acid having Functional group (A-2), cysteine is preferred.

Examples of a cyclization method using the amino acid having Functional group (A-1) and the amino acid having Functional group (A-2) include methods described in Kawakami, T. et al., Nature Chemical Biology 5, 888-890 (2009); Yamagishi, Y. et al., ChemBioChem 10, 1469-1472 (2009); Sako, Y. et al., Journal of American Chemical Society 130, 7932-7934 (2008); Goto, Y. et al., ACS Chemical Biology 3, 120-129 (2008); Kawakami T. et al, Chemistry & Biology 15, 32-42 (2008); and WO2008/117833.

Examples of an amino acid having Functional group (B-1) include propargylglycine, homopropargylglycine, 2-amino-6-heptynoic acid, 2-amino-7-octynoic acid, and 2-amino-8-nonynoic acid.

A 4-pentynoylated or 5-hexynoylated amino acid may also be used.

Examples of the 4-pentynoylated amino acid include N-(4-pentenoyl)-L-alanine, N-(4-pentenoyl)-L-phenylalanine, N-(4-pentenoyl)-L-tyrosine, N-(4-pentenoyl)-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophan, β-N-(4-pentenoyl)-L-diaminopropanoic acid, γ-N-(4-pentenoyl)-L-diaminobutyric acid, σ-N-(4-pentenoyl)-L-ornithine, and ε-N-(4-pentenoyl)-L-lysine, and D-amino acid derivatives corresponding thereto.

Examples of the 5-hexynoylated amino acid include amino acids obtained by substituting the 4-pentynoyl group of the compounds exemplified as the 4-pentynoylated amino acid by a 5-hexynoyl group.

Examples of an amino acid having Functional group (B-2) include azidoalanine, 2-amino-4-azidobutanoic acid, azidoptonorvaline, azidonorleucine, 2-amino-7-azidoheptanoic acid, and 2-amino-8-azidooctanoic acid.

In addition, azidoacetylated or 3-azidopentanoylated amino acids may also be used.

Examples of the azidoacetylated amino acids include N-azidoacetyl-L-alanine, N-azidoacetyl-L-phenylalanine, N-azidoacetyl-L-tyrosine, N-azidoacetyl-L-tryptophan, N-3-(4-pentynoylamido)benzoyl-L-phenylalanine, N-3-(4-pentynoylamido)benzoyl-L-tyrosine, N-3-(4-pentynoylamido)benzoyl-L-tryptophan, β-N-azidoacetyl-L-diaminopropanoic acid, γ-N-azidoacetyl-L-diaminobutyric acid, σ-N-azidoacetyl-L-ornithine, and ε-N-azidoacetyl-L-lysine, and D-amino acid derivatives corresponding thereto.

Examples of the 3-azidopentanoylated amino acids include amino acids obtained by substituting the azidoacetyl group of the compounds exemplified as the azidoacetylated amino acids by a 3-azidopentanoyl group.

Examples of a cyclization method using the amino acid having Functional group (B-1) and the amino acid having Functional group (B-2) include those described in Sako, Y et al., Journal of American Chemical Society 130, 7932-7934 (2008) and WO2008/117833.

Examples of an amino acid having Functional group (C-1) include N-(4-aminomethyl-benzoyl)-phenylalanine (AMBF) and 3-aminomethyltyrosine.

Examples of an amino acid having Functional group (C-2) include 5-hydroxytryptophan (WOH).

Examples of a cyclization method using the amino acid having Functional group (C-1) and the amino acid having Functional group (C-2) include those described in Yamagishi, Y. et al., ChemBioChem 10, 1469-1472 (2009) and WO2008/117833.

Examples of an amino acid having Functional group (D-1) include 2-amino-6-chloro-hexynoic acid, 2-amino-7-chloro-heptynoic acid, and 2-amino-8-chloro-octynoic acid.

Examples of an amino acid having Functional group (D-2) include cysteine, homocysteine, mercaptonorvaline, mercaptonorleucine, 2-amino-7-mercaptoheptanoic acid, and 2-amino-8-mercaptooctanoic acid.

Examples of a cyclization method using the amino acid having Functional group (D-1) and the amino acid having Functional group (D-2) include that described in WO2012/074129.

### Examples of an amino acid (E-1) include

N-3-chloromethylbenzoyl-L-phenylalanine, N-3-chloromethylbenzoyl-L-tyrosine, and N-3-chloromethylbenzoyl-L-tryptophane, and D-amino acid derivatives corresponding thereto.

Examples of an amino acid (E-2) include cysteine, homocysteine, mercaptonorvaline, mercaptonorleucine, 2-amino-7-mercaptoheptanoic acid, and 2-amino-8-mercaptooctanoic acid.

A cyclization method using the amino acid having Functional group (E-1) and the amino acid having Functional group (E-2) can be carried out with reference to, for example, the cyclization method using (A-1) and (A-2) or the cyclization method using (D-1) and (D-2).

Specific examples of the cyclic peptide of the present invention include that represented by the following formula (12):

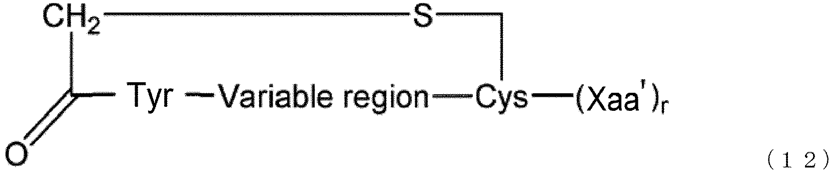

In the formula (12), Tyr may be either a D-form or L-form; and S means a thiol group of Cys. In the present specification and drawing attached thereto, the structure represented by -CH₂-CO- in the aforesaid structure is also described as Ac.

In the formula (12), a portion indicated by "Variable region" is any of the structures containing at least one structure represented by the formula (1).

Xaa' and r have the same meanings as described above and preferred aspects of them are also similar to those described above.

In the aforesaid formula (12), the "Variable region" is preferably represented by the following formula (13):

-(Z⁴)p-Xaa¹-(Xaa²)ₙ₁-Xaa³-Xaa⁴-(Z⁵)_{q}- (13)

In the formula (13), Xaa¹, Xaa², Xaa³, Xaa⁴, n1, Z⁴, Z⁵, p, and q have the same meanings as Xaa¹, Xaa², Xaa³, Xaa⁴, n1, Z⁴, Z⁵, p, and q described above in the structures represented by the formula (1) and the formula (11) and may have a structure having any combination of the preferred aspects described as Xaa¹, Xaa², Xaa³, Xaa⁴, n1, Z⁴, Z⁵, p, and q described in the structures represented by the formula (1) and the formula (11).

In the formula (13), p is preferably any integer of 0 to 3, more preferably any integer of 0 to 2, still more preferably 0 or 1.

In the formula (13), q is preferably any integer of 0 to 3, more preferably any integer of 0 to 2, still more preferably 0 or 1.

In the formula (13), when p and/or q is 1 or more, Z⁴ and Z⁵ may each independently contain an amino acid having a branched side chain such as Leu, Val, or Ile, an amino acid having a hydroxyl group in the side chain such as Ser or Thr, a basic amino acid, or an aromatic amino acid, preferably Leu or Ser.

Preferred examples of the "Variable region" in the formula (12) include structures represented by the following formulas (13) to (16).

-(Z⁴)ₚ-Xaa¹-(Xaa²)ₙ₁-Xaa³-Xaa⁴-(Z⁵)_{q}- (13)

-(Z⁴)ₚ-Arg-(Xaa²)ₙ₁-Phe-Trp-(Z⁵)_{q}- (14)

-Leu-Arg-(Xaa²)ₙ₁-Phe-Trp- (15)

-Arg-(Xaa²)ₙ₁-Phe-Trp-Ser- (16)

In the formulas (13) to (16), Xaa¹, Xaa², Xaa³, Xaa⁴, n1, Z⁴, Z⁵, p, and q have the same meanings as Xaa¹, Xaa², Xaa³, Xaa⁴, n1, Z⁴, Z⁵, p, and q described above in the structures represented by the aforesaid formula (1) and the formula (11) and may be a structure having any combination of the preferred aspects described as Xaa¹, Xaa², Xaa³, Xaa⁴, n1, Z⁴, Z⁵, p, and q described in the structures represented by the formula (1) and the formula (11).

Specific preferred examples of the cyclic peptide represented by the aforesaid formula (12) include those having any of the structures represented by the following formulas (3) to (8). In the following formulas (3) to (8), a linear structure portion corresponding to (Xaa')ᵣ to be bound to Cys of the aforesaid formula (12) is omitted. In the following formulas (3) to (8), therefore, (Xaa')ᵣ may bind to C (Cys) bound to S (Xaa' and r have the same meanings as those of the formula (12)).

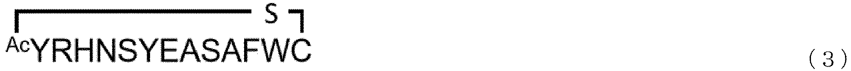

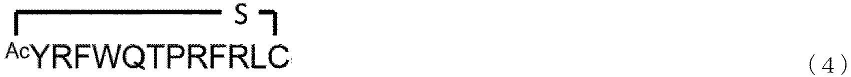

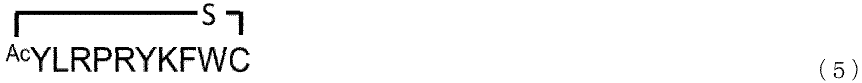

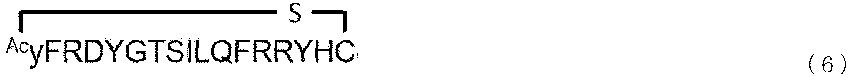

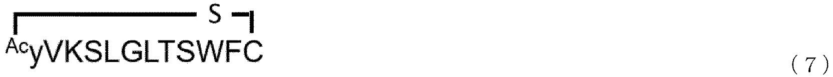

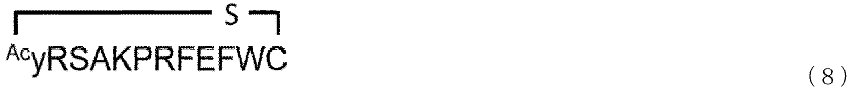

In the formulas (3) to (8), Ac is -CH₂-CO-, Y is L-tyrosine, and y is D-tyrosine. The amino acid sequence in the structure of the formulas (3) to (5) will be indicated in SEQ ID NOS: 20 to 22.

### [Production method of cyclic peptide]

The cyclic peptide of the present invention can be preferably produced by the translation synthesis method in a cell-free translation system.

It can be prepared by preparing a nucleic acid encoding the cyclic peptide and translating the nucleic acid in a cell-free translation system. The nucleic acid encoding the cyclic peptide can be designed as needed by those skilled in the art by using a genetic code used in vivo translation system, a reprogrammed genetic code, or a combination of them. The nucleic acid may be either DNA or RNA.

In accordance with the method using a cell-free translation system, an unnatural amino acid as well as a natural amino acid can be introduced efficiently into a peptide by using tRNA aminoacylated with an unnatural amino acid. For example, tRNA having any anticodon can be aminoacylated with any natural or unnatural amino acid by using the artificial aminoacyl-tRNA synthetase Flexizyme developed by the present inventors. By using this technology, therefore, it is possible to reprogram a genetic code made of an mRNA triplet so that it encodes an amino acid different from that in a vivo translation system (WO2008/059823).

### [Peptide complex]

The peptide complex and pharmacologically acceptable salt thereof according to the present invention contains two cyclic peptides of the present invention, and one of the cyclic peptides is linked to the other cyclic peptide via a linker.

As shown in Fig. 1, BDNF is known to bind to the extracellular domain of TrkB, cause the homodimerization of TrkB, and activate downstream signaling by autophosphorylation of TrkB.

The peptide complex and pharmacologically acceptable salt thereof according to the present invention have the structure as described above, so that the respective cyclic peptides in the peptide complex are thought to bind to the extracellular domain of TrkB and cause the homodimerization of TrkB. The peptide complex and pharmacologically acceptable salt thereof according to the present invention are therefore thought to activate the downstream signaling by autophosphorylation of TrkB. As a result, the peptide complex and pharmacologically acceptable salt thereof according to the present invention are thought to stimulate various cell responses such as neurite branching, survival of neurons, and axonal outgrowth. The peptide complex of the present invention therefore preferably induces phosphorylation of TrkB.

When the peptide complex or pharmacologically acceptable salt thereof according to the present invention binds to the extracellular domain of TrkB and causes homodimerization of TrkB and then autophosphorylation of TrkB, the peptide complex induces association of SH2 (src homology-type 2) linker proteins such as shc (src homology domain containing protein) and IRS1/2 (insulin receptor substrate 1/2), leading to activation of PI3K and ERK signaling pathway.

The peptide complex of the present invention is not particularly limited as long as it contains two cyclic peptides of the present invention and one of the cyclic peptides is linked to the other cyclic peptide via a linker. These two cyclic peptides may be the same or different from each other. From the standpoint of further promoting homodimerization of TrkB, the two cyclic peptides in the peptide complex are preferably identical.

The linker for linking the two cyclic peptides of the peptide complex is not particularly limited and a linker having a known structure as a linker which links peptides to each other in the peptide synthesis field can be used. A linker having a length of 20 Å or more and 200 Å or less is preferably used. Such an aspect tends to promote the homodimerization of TrkB further. From the similar standpoint, the length of a linker is more preferably 30 Å or more and 150 Å or less, still more preferably 40 Å or more and 100 Å or less.

Non-limiting examples of the linker for linking the two cyclic peptides of the peptide complex include polymers such as polyethylene glycol (PEG), peptides, nucleic acids, and sugars, and combinations thereof.

The PEG-containing linker is, for example, a small molecule having two or more functional groups to which two PEGs bind, respectively. Non-limiting examples include carboxylic acid-modified PEG bound to the amino group of 2,3-diaminopropionic acid.

In the PEG-containing linker, the polymerization number of ethylene glycol may be 2 or more and 20 or less, 3 or more and 15 or less, or 4 or more and 10 or less.

### [Production method of peptide complex]

The peptide complex of the present invention may be produced by linking the cyclic peptides of the present invention obtained as described above via an appropriate linker.

A step of linking the cyclic peptides of the present invention by a linker can be carried out by those skilled in the art by a known method of linking peptides with a linker or a method based thereon, depending on the kind of the linker.

For example, when the linker is a linker containing the aforesaid PEG, two cyclic peptides of the present invention may be linked via the linker by the method shown in Fig. 2.

When the linker is a peptide, two nucleic acids encoding the cyclic peptides of the present invention may be linked to a nucleic acid encoding the linker peptide to express a fused protein of the cyclic peptides of the present invention and the linker peptide.

The cyclic peptide and pharmacologically acceptable salt thereof according to the present invention have high affinity for TrkB, can strongly bind to TrkB, and can act as a TrkB agonist.

Moreover, the peptide complex containing the cyclic peptides of the present invention binds to the extracellular domain of TrkB, causes homodimerization of TrkB, and thereby selectively induces downstream TrkB signaling. The peptide complex of the present invention therefore stimulates various cell responses such as neurite branching, survival of neurons, and axonal outgrowth.

The cyclic peptide of the present invention has low pharmacokinetic properties, particularly low cellular permeability, different from a small-molecule TrkB agonist conventionally studied, making it possible to provide a method for treating and researching neurological diseases using an approach different from that of the conventional method.

### [Pharmaceutical composition]

The pharmaceutical composition of the present invention contains at least one selected from the group consisting of the cyclic peptides and the pharmacologically acceptable salts thereof, and the peptide complexes and the pharmacologically acceptable salts thereof. The pharmaceutical composition of the present invention is therefore used preferably for the treatment or prevention of neurodegenerative diseases, neurodevelopmental disorders, or neuropsychiatric diseases.

The pharmaceutical composition may have, as an effective ingredient, at least one selected from the group consisting of the cyclic peptides and the pharmacologically acceptable salts thereof, and the peptide complexes and the pharmacologically acceptable salts thereof as is or it may be formulated together with a pharmaceutically acceptable additive and the like.

Examples of the dosage form of the pharmaceutical formulation include solutions (for example, injections), dispersions, suspensions, tablets, pills, powdered drug, suppositories, powders, fine granules, granules, capsules, syrups, troches, inhalants, ointments, ophthalmic formulations, nasal formulations, ear formulations, and cataplasms.

The pharmaceutical composition may be formulated in a conventional manner by using, for example, an additive such as excipient, binder, disintegrant, lubricant, dissolving agent, solubilizing agent, colorant, taste/odor corrigent, stabilizer, emulsifier, absorption promoter, surfactant, pH regulator, antiseptic, humectant, dispersing agent, or antioxidant as needed.

The additive used for the formulation is not particularly limited and examples include purified water, saline, phosphate buffer, pharmaceutically acceptable organic solvents such as dextrose, glycerol, and ethanol, animal or vegetable oils, lactose, mannitol, glucose, sorbitol, crystalline cellulose, hydroxypropyl cellulose, starch, corn starch, silicic anhydride, magnesium aluminum silicate, collagen, polyvinyl alcohol, polyvinylpyrrolidone, carboxy vinyl polymer, carboxymethylcellulose sodium, sodium polyacrylate, sodium alginate, water-soluble dextran, carboxymethyl starch sodium, pectin, methyl cellulose, ethyl cellulose, xanthan gum, gum arabic, tragacanth, casein, agar, polyethylene glycol, diglycerin, glycerin, propylene glycol, petrolatum, paraffin, octyl dodecyl myristate, isopropyl myristate, higher alcohol, stearyl alcohol, stearic acid, and human serum albumin.

As an absorption promoter in transmucosal absorption, surfactants such as polyoxyethylene lauryl ethers, sodium lauryl sulfate, and saponin; bile salts such as glycocholate, deoxycholate, and taurocholate; chelating agents such as EDTA and salicylic acid; fatty acids such as caproic acid, capric acid, lauric acid, oleic acid, linoleic acid, and mixed micelle; enamine derivatives, N-acylcollagen peptide, N-acylamino acid, cyclodextrines, chitosans, and nitric oxide donors may be used.

The tablets or pills may be sugar-, gastric-, or enteric-coated.

The solutions may contain distilled water for injection, physiological saline, propylene glycol, polyethylene glycol, a vegetable oil, an alcohol, or the like. The solutions may further contain a humectant, an emulsifier, a dispersant, a stabilizer, a dissolving agent, a solubilizing agent, an antiseptic, or the like.

The present invention also provides a method of treating or preventing a disease of a patient by administering the pharmaceutical composition to the patient who needs it. The disease may be neurodegenerative diseases, neurodevelopmental disorders, or neuropsychiatric diseases.

The dose of the pharmaceutical composition of the present invention may be determined as needed by those skilled in the art, depending on the symptom, age, sex, weight, and difference in sensitivity of patients who require the pharmaceutical composition, the administration method, the administration interval, the type of the formulation, or the like.

The patient is a mammal, preferably a human being.

### Examples

The present invention will hereinafter be described more specifically by Examples and Comparative Examples. The present invention is no way limited by the following Examples.

### [Preparation of cyclic peptide]

The cyclic peptide was prepared by the following procedure.

### (Preparation of cyclic peptide library and selection of cyclic peptide that binds to hTrkB)

Two thioether-cyclic peptide libraries (L-library and D-library) were constructed using the flexible in vitro translation (FIT) system. By the previously reported method, N-(2-chloroacetyl)-L-tyrosine (L-ClAcTyr) and N-(2-chloroacetyl)-D-tyrosine (D-ClAcTyr) were introduced into the L-library and the D-library, respectively (Kawakami, T.; Murakami, H.; Suga, H., Messenger RNA-programmed incorporation of multiple N-methyl-amino acids into linear and cyclic peptides. Chem Biol 2008, 15 (1), 32-42.; Morimoto, J.; Hayashi, Y; Suga, H., Discovery of macrocyclic peptides armed with a mechanism-based warhead: isoform-selective inhibition of human deacetylase SIRT2. Angew Chem Int Ed Engl 2012, 51 (14), 3423-7.; Goto, Y; Katoh, T.; Suga, H., Flexizymes for genetic code reprogramming. Nat Protoc 2011, 6 (6), 779-90.; Hayashi, Y; Morimoto, J.; Suga, H., In vitro selection of anti-Akt2 thioether-macrocyclic peptides leading to isoform-selective inhibitors. ACS Chem Biol 2012, 7 (3), 607-13.).

mRNA libraries corresponding to the thioether-cyclic peptide libraries were designed so that they had, in order, an AUG start codon encoding L-ClAcTyr or D-ClAcTyr, 8 to 15 NNK random codons (N is G, C, A, or U and K is G or U) encoding a random proteinogenic amino acid residue, and a UGC codon encoding Cys. The in vitro translation was followed by spontaneous formation of a thioether bond between the acetyl chloride group of the Tyr residue at the N terminal and the sulfhydryl group (thiol group) of the downstream cysteine residue.

The selections (which may also be called "affinity selection") of a cyclic peptide that binds to human TrkB (hTrkB) were carried out using the RaPID (random non-standard peptides integrated discovery) system independently for each of the L-library and D-library (Fig. 3) (Hayashi, Y; Morimoto, J.; Suga, H., In vitro selection of anti-Akt2 thioether-macrocyclic peptides leading to isoform-selective inhibitors. ACS Chem Biol 2012, 7 (3), 607-13.; Hipolito, C.J. & Suga, H. Ribosomal production and in vitro selection of natural product-like peptidomimetics: the FIT and RaPID systems. Curr Opin Chem Biol. 16, 196-203, 2012).

More specifically, the preparation of cyclic peptide libraries and selection of cyclic peptides that binds to hTrkB were performed in the following manners.

The mRNA library, ClAc-L-Tyr-tRNA^{fMet}_{CAU}, and ClAc-D-Tyr-tRNA^{fmet}_{CAU} were prepared as reported previously (Hayashi, Y; Morimoto, J.; Suga, H., In vitro selection of anti-Akt2 thioether-macrocyclic peptides leading to isoform-selective inhibitors. ACS Chem Biol 2012, 7 (3), 607-13.; Hipolito, C.J. & Suga, H. Ribosomal production and in vitro selection of natural product-like peptidomimetics: the FIT and RaPID systems. Curr Opin Chem Biol. 16, 196-203, 2012).

The 4 µM mRNA libraries were linked to a 1.5 µM puromycin linker at 25°C for 30 minutes by using a T4 RNA ligase. Puromycin-linked DNA bound to the 3'-end constant region of the mRNA library. After purification by phenol-chloroform extraction and ethanol precipitation, translation was carried out using the resulting 1.2 µM mRNA-puromycin conjugate and 25 µM ClAc-L-Tyr-tRNA^{fmet}_{CAU} or ClAc-D-Tyr-tRNA^{fMet}_{CAU} at 37°C for 30 minutes in a methionine-deficient FIT system. Then, the reaction products were incubated further at 25°C for 12 minutes to promote the formation of mRNA-peptide conjugates. The products were incubated further at 37°C for 30 minutes in the presence of 20 mM EDTA. Then, the reverse transcription of the product was performed at 42°C for one hour with RNase-H minus reverse transcriptase (Promega) to tag an mRNA-cDNA hybrid with the cyclic peptide. Then, the buffer solution was replaced by that containing 50 mM Tris-HCl (pH 7.4), 150 mM NaCl, and 0.05 vol% tween-20.

Next, the peptide-mRNA/cDNA conjugate libraries thus obtained were subjected to affinity selection. First, as control selection, the peptide-mRNA/cDNA conjugate libraries were passed three times through His pulldown Dynabeads (Life Technologies) (each passage for 10 minutes under the condition of 4°C). The above process is called "pre-clearance" or negative selection and is performed to remove undesired binding to beads. Next, the pre-cleared peptide-mRNA/cDNA conjugate libraries were subjected to affinity selection under the condition of 4°C for 30 minutes using the beads having a 200 nM TrkB extracellular domain fixed thereto. The process is called "positive selection".

By heating at 95°C for 5 minutes, the cDNAs were eluted from the beads and a portion of the eluted cDNAs was evaluated by quantitative PCR using Sybr Green I and thermal cycler (LightCycler (Roche)). The remaining portion of the cDNAs was amplified by PCR and used for the transcription of the mRNA library used for affinity selection in the next round.

One round consisted of preparation of a library, pre-clearance, and positive selection, and the round was performed 5 times. After 5-times round, significant enrichment of the cDNAs were observed (compared with the positive selection performed without fixing the extracellular domain of TrkB on the beads) (Fig. 4). The collected cDNAs were amplified by PCR, purified using a column (NucleoSpin (Machery-Nagel)), and sequenced using a high through-put sequencer (MiSeq (Illumina)). Data analysis was performed using a software (CLC Sequence Viewer 7 (Qiagen)).

Examples of the structure of a peptide which forms the cyclic peptide that binds to hTrkB and was selected by the RaPID system are shown in the following Tables 2 and 3.

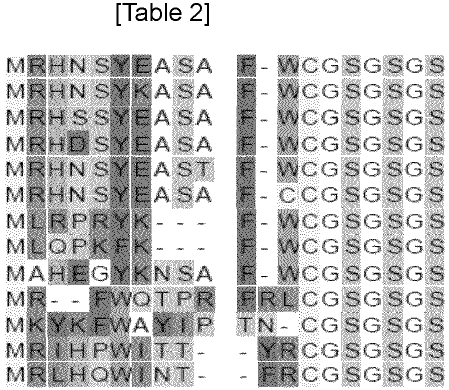

In the sequences of Table 2, M at the leftmost end is L-ClAcTyr and the other English alphabet indicates an amino acid representing an amino acid according to one letter notation. In the peptides shown in Table 2, the leftmost end L-ClAcTyr and the downstream first cysteine residue formed a thioether bond therebetween. The GSGSGS sequence downstream in each peptide is a linear structure that binds to the cyclic structure and it is not always necessary. The peptides shown in Table 2 correspond to, from the top, SEQ ID NOS: 1 to 13, respectively.

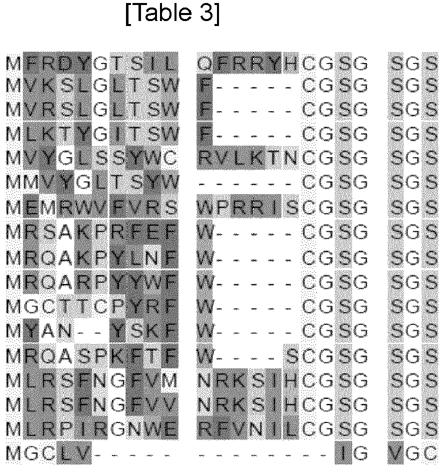

In the sequence of Table 3, M at the leftmost end is D-ClAcTyr and the other English alphabet indicates an amino acid representing an amino acid according to one letter notation. In the peptides shown in Table 3, the leftmost end D-ClAcTyr and the downstream first cysteine residue formed a thioether bond therebetween. The GSGSGS sequence downstream in each peptide is a linear structure that binds to the cyclic structure and it is not always necessary.

### (Chemical synthesis of cyclic peptide)

From the sequenced cyclic peptides, three ones with the highest proportion in each of the L-library and the D-library were produced by chemical synthesis. Described specifically, cyclic peptides were synthesized by the Fmoc solid phase peptide synthesis (SPPS) by using a Syro Wave automated peptide synthesizer (Biotage) based on the known method (Ito, K. et al. Artificial human Met agonists based on macrocycle scaffolds. Nature Communications 6, 6372, 2015).

More specifically, after automated synthesis of each peptide, a chloroacetyl group for cyclization was bound to the N-terminal amide group of the resulting product. The peptides were cleaved from the solid phase by using a solution of 92.5% trifluoroacetic acid (TFA), 2.5% water, 2.5% triisopropylsilane, and 2.5% ethanedithiol and precipitated in diethyl ether. For performing a cyclization reaction, the peptide pellets were dissolved in 10 ml of triethylamine containing DMSO and incubated at 25°C for 1 hour. The peptide suspensions were then acidified by the addition of TFA thereto and the cyclization reaction was stopped.

Then, the peptides were purified by reversed-phase HPLC (RP-HPLC) under linear gradient conditions by using a Prominence HPLC system (Shimazu). Mobile phase A containing water containing 0.1 % TFA was mixed with Mobile phase B containing acetonitrile containing 0.1 % TFA to achieve gradient. Purified peptides were lyophilized in vacuo and were subjected to MALDI-TOF mass analysis using AutoFlex II (Bruker Daltonics) to confirm their molecular weight.

The structure of the cyclic peptides binding to hTrkB, which were selected by the RaPID system and chemically synthesized, is shown in the following Table 4.

**[Table 4]**

| | L-library | Abundance (%) | | D-library | Abundance (%) |
|---|---|---|---|---|---|
| TrbL1 | 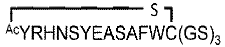 | 18 | TrbD1 | 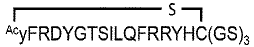 | 19 |
| TrbL2 | 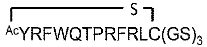 | 15 | TrbD2 | 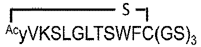 | 14 |
| TrbL3 | 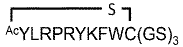 | 9 | TrbD3 | 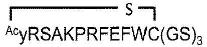 | 7 |

In the above table, Ac is -CH₂-CO-, Y is L-tyrosine, and y means D-tyrosine. The term "Abundance" represents the percent of the peptide in the total L-peptide or D-peptide obtained by the affinity selection and a higher value suggests that the binding force of the peptide to TrkB is stronger.

### [Evaluation of cyclic peptide]

The cyclic peptides TrbL1 to 3 and TrbD1 to 3 obtained as described above were subjected to an activation test.

### (Evaluation of affinity for hTrkB)

With respect to the binding kinetics (binding affinity (dissociation constant) K_{D}) of the cyclic peptides towards the extracellular domain of hTrkA, hTrkC, and hTrkB (which will hereinafter be called "hTrkA-C proteins", collectively) immobilized on a sensor chip via a His6-tag, quantitative measurements making use of surface plasmon resonance (SPR) were carried out to evaluate the binding intensity and selectivity of the cyclic peptide to hTrkB, compared with that to hTrkA and hTrkC.

The binding kinetics of each peptide towards the hTrkA-C proteins were determined using Biacore T200 (GE Healthcare). The buffer used was HBS-P+ (10 mM HEPES, 150 mM NaCl, and 0.05 vol% surfactant P20, pH 7.4) containing 0.1 vol% DMSO and 1 mM b-dodecyl-maltoside. 100 nM hTrkA-C proteins were immobilized on a Ni²⁺ treated NTA chip in accordance with the manufacturer's instructions (baseline response 1000 to 3000 refraction units). Peptides were injected as an analyte at a flow rate of 60 mL min⁻¹ and binding kinetics was modelled using a 1:1 binding model.

The SPR responses of the cyclic peptides (TrbL1 to 3 and TrbD1 to 3) to the hTrkB is shown in Fig. 5 and the binding affinity K_{D} for the hTrkA-C proteins is shown in Table 5.

**[Table 5]**

| **Monomer** | **TrkA** | **TrkB** | **TrkC** |
|---|---|---|---|
| **TrbL1** | N.B. | 26 | N.B. |
| **TrbL2** | 100 | 70 | N.B. |
| **TrbL3** | N.B. | **1.5** | N.B. |
| **TrbD1** | 150 | 6 | N.B. |
| **TrbD2** | N.B. | 18 | N.B. |
| **TrbD3** | 123 | 15 | N.B. |

In the table, the term "N.B." represents non-binding. In other words, it means that no SPR response was detected within a range of the hTrkA-C proteins from 1 to 1000 nM.

The K_{D} values of all the peptides (TrbL1 to 3 and TrbD1 to 3) for hTrkB were 1.5 to 70 nM and all the peptides (TrbL1 to 3 and TrbD1 to 3) showed strong affinity for hTrkB. Among them, three peptides (TrbL1, TrbL3, and TrbD2) showed strong selectivity for hTrkB and did not show an SPR signal for hTrkA and hTrkC. Particularly, TrbL3 had a K_{D} of 1.5 nM for hTrkB and thus, it had very high hTrkB selectivity.

### [Preparation of peptide complex]

With respect to the cyclic peptides TrbL1 to 3 and TrbD1 to 3 obtained as described above, peptide complexes (homodimeric peptides) containing these cyclic peptides were prepared in the following procedure.

Peptide complexes (homodimeric peptides) were obtained by dimerizing TrbL1 to 3 and TrbD1 to 3 via a linker and the resulting peptide complexes corresponding to TrbL1 to 3 and TrbD1 to 3 will hereinafter be called DiTrbL1 to 3 and DiTrbD1 to 3, respectively.

All the dimeric peptides were synthesized, as previously reported, using conventional Fmoc-based SPPS on a Syro I automated peptide synthesizer (Biotage) (Bashiruddin, N. K.; Matsunaga, Y; Nagano, M.; Takagi, J.; Suga, H., Facile Synthesis of Dimeric Thioether-Macrocyclic Peptides with Antibody-like Affinity against Plexin-B1. Bioconjug Chem 2018, 29 (6), 1847-1851.) (Fig. 2).

In Fig. 2, coupling of Fmoc-DAP (Fmoc), Fmoc-NH-(PEG)₅-COOH linker, and other Fmoc-protected amino acids were performed on DMF engorged NovaPEG Rink Amide resins in the presence of 0.5 M Fmoc-protected amino acid, 0.5 M HBTU/HOBt, and 0.5 M DIPEA in DMF. After three programmed washes with DMF, Fmoc-deprotection was performed by a 30 min RT treatment with 40% piperidine in DMF. Double coupling was used for all steps. After all the amino acids were coupled, 0.2 M chloroacetyl-NHS in DMF was used to add a chloroacetyl group to the N terminal of each peptide. The step was repeated twice, followed by three DMF washes, six DCM washes, and vacuum desiccation. The desiccated peptide-conjugated resins were then subjected to global deprotection and treated in a pre-chilled TFA/H₂O/EDT/TIS (92.5:2.5:2.5:2.5) mixture for one hour at room temperature to cleave a dimeric peptide from the resins. The deprotection solutions were precipitated with chilled diethyl ether and the pellets were dried in fume hoods.

The resulting pellets were dissolved in DMSO containing 0.1% TFA, followed by the addition of TEA until the solution was basic (confirmed using H₂O and pH indicator paper) to induce cyclization of the peptides. After cyclization for 30 minutes at room temperature under basic conditions, the mass of the peptides and completion of the cyclization were confirmed by a MALDI-TOF mass analyzer (AutoFlex II (Bruker Daltonics)).

After the mass of the peptides and completion of the cyclization were confirmed, the peptide solutions were brought back to acidic conditions with TFA (confirmed using H₂O and pH indicator paper) and were subjected to reverse-phase HPLC (RP-HPLC) using a Prominence HPLC system (Shimazu) connected to a Chromolith ODC column to purify the peptides. The flow rate was set at 25 mL/min and gradient was set at 10 to 70% MeCN (containing 0.1% TFA) in deionized H₂O (containing 0.1% TFA) for 32 minutes.

As a result, DiTrbL1 to 3 and DiTrbD1 to 3, that is, peptide complexes (homodimeric peptides) obtained by dimerization of TrbL1 to 3 and TrbD1 to 3, respectively, via a PEG₅-DAP-PEG₅ linker (having a length of 58 Å) were obtained.

### [Evaluation method of peptide complex]

The peptide complexes obtained as described above were subjected to various activity tests. The details of the various activity test are as follows.

### (Surface plasmon resonance (SPR): evaluation of binding kinetics (binding affinity K_{D})

In a manner similar to the aforesaid method used for the evaluation of the binding kinetics (binding affinity K_{D}) of the cyclic peptides except for the use of the peptide complexes (DiTrbL1 to 3 and DiTrbD1 to 3) instead of the cyclic peptides (TrbL1 to 3 and TrbD1 to 3), the binding kinetics (binding affinity K_{D}) of the peptide complexes (DiTrbL1 to 3 and DiTrbD1 to 3) were evaluated.

### (West blotting method)

DIV6 (6 days in vitro) hippocampal neurons were treated for 15 minutes with DMSO, BDNF, or the peptide complex (DiTrbL3). ANA-12 (100 µM) was added 30 minutes before the treatment with BDNF or the peptide.

After washing with 0.1 M PBS twice, cells were lysed in a radio-immunoprecipitation assay (RIPA) buffer containing 50 mM Tris-HCl (pH 7.4), 150 mM NaCl, 2 mM EDTA, 1% Nonidet P-40, 0.5% Na-deoxycholate, 0.1% SDS, 50 mM NaF, 0.2 mM Na₃VO₄, 10 µg/mL aprotinin, 10 µg/mL leupeptin, and 1 mM phenylmethylsulfonyl fluoride on ice for 30 minutes. After ultrasonic treatment and centrifugation at 20,000 ×g for 30 minutes, the supernatant was subjected to SDS-PAGE. Protein amount was quantified using Bicinchoninic Acid Kit (BCA-1, Sigma).

The cell lysate (protein amount: 20 µg in P-TrkB, 10 µg in TrkB, and 5 µg in Akt and Erk) was separated using 8% polyacrylamide gel and transferred to a polyvinylidene difluoride membrane (Merck Millipore, Massachusetts, USA). The resulting membrane was blocked for 1 hour with a blocking buffer (5% bovine serum albumin (BSA) tris-buffered physiological saline (TBST) containing 0.05% tween-20), followed by incubation overnight at 4°C in a blocking buffer containing a primary antibody (rabbit anti-TrkB antibody, 1:1,000 dilution, Cat#4603; rabbit anti-phosphorylated Trk antibody, 1:1,000 dilution, Cat#9141; mouse anti-pan-Akt antibody, 1:2,000 dilution, Cat#2920; rabbit anti-phosphorylated Akt (S473) antibody, 1:2,000 dilution, Cat#4060; rabbit anti-Erk1/2 antibody, 1:2,000 dilution, Cat#4695; mouse anti-phosphorylated Erk1/2 antibody, 1:2,000 dilution, Cat#9106 (Cell Signaling Technology)). After washing with TBST for 10 minutes four times, the membrane was incubated with a secondary antibody (peroxidase-conjugated goat anti-rabbit IgG antibody, 1:10,000 dilution, Cat#111-035-003 (Jackson Immuno Research); peroxidase-conjugated sheep anti-mouse IgG antibody, 1:10,000 dilution, Cat#NA9310 (GE Healthcare)) for one hour at room temperature.

Finally, after the resulting membrane was washed with TBST, an immune-positive bands were detected by a chemiluminescence method using a Luminata Crescendo HPR substrate (Merck Millipore). The relative phosphorylation level of each kinase was calculated as a ratio of phosphorylated kinase/all kinases.

### (Phospho-RTK array evaluation)

DIV6 hippocampal neurons were incubated with DMSO, BDNF (0.1 nM) or DiTrbL3 (1 nM) in 5% CO₂ at 37°C for 15 minutes. ANA-12 (100 µM) was added 30 minutes before the DiTrbL3 treatment. Comprehensive study of the phosphorylated tyrosine kinase receptor was conducted using a phospho-RTK array in accordance with the manufacturer's protocol (Proteome Profiler mouse Phospho-RTK Array Kit, ARY014, R&D Systems). The phosphorylation level was evaluated by pixel density and quantified by Image J Software.

### (RNA extraction and qRT-PCR)

After DIV6 primary cortical neurons were treated with BDNF or the peptide complex (DiTrbL3) for one hour, total RNA was extracted from the primary neurons using SV Total RNA Isolation System (Z3105, Promega). The total RNA was quantitatively and qualitatively verified by a NanoDrop spectrometer (Thermo Fisher Scientific) and electrophoresis in a formaldehyde gel.

Next, 500 ng of the total RNA was reverse-transcribed using Super Script II (Thermo Fisher Scientific). Quantitative PCR was carried out using a SYBR Green real-time PCR system by the previously reported method (Akashi, M.; Takumi, T., The orphan nuclear receptor RORalpha regulates circadian transcription of the mammalian core-clock Bmal1. Nat Struct Mol Biol 2005, 12 (5), 441-8.). A Gapdh gene was used as an internal control. The following primer sequences were respectively used: Gapdh (Forward: 5'-ACGGAAGCTCACTCAGGCATGCATGCCTT-3' and reverse: 5'-CATGAGGTCCCCCCACCCTGTTTGCTGCTG-3') (represented by SEQ ID NOS: 14 and 15, respectively), c-fos (Forward: 5'-AGTTTTCTCTACTACTACTACATCC-3' and reverse: 5'-AAAAGTTGGCACTAGACGACGACGACGAC-3') (represented by SEQ ID NOS: 16 and 17, respectively), and Arc (Forward: 5'-GGTGAGCTGACTGACCAAAT-3' and reverse: 5'-TTCACTGGTATCATCACTGATCACTGCTGCTG-3')(represented by SEQ ID NOS: 18 and 19, respectively).

### (Immunocytochemistry for evaluation of axonal outgrowth)

Primary hippocampal neurons at DIVa were treated with DMSO, BDNF, or DiTrbL3 peptide and subjected to immunocytochemical analysis at DIV3.

The cells were washed twice with 0.1 M phosphate-buffered physiological saline (PBS) and immobilized for 15 minutes with 2% paraformaldehyde (PFA)/4% sucrose (in 0.1 M PBS). After washing three times with PBS, the cells were subjected to permeabilization for 3 minutes with PBS (PBST) containing 0.2% Triton-X and incubated for one hour with a blocking solution (PBST containing 5% normal goat serum) to prevent non-specific binding. Then, the cells were incubated overnight at 4°C with a primary antibody diluted with the blocking solution (rabbit anti-Tau polyclonal antibody, 1:1,000 dilution; cat#ab64193 (abcam); mouse anti-MAP2 monoclonal antibody, 1:1,000 dilution, M4403 (Sigma)). After washing of the cells with PBST for 10 minutes was performed four times, they were incubated at room temperature for 2 hours with a fluorescently-labeled secondary antibody diluted with the block solution (Alexa Fluor 488-labeled goat anti-mouse IgG antibody, 1:1,000 dilution, A-11029; Alexa Fluor 568-labeled goat anti-rabbit IgG antibody, 1:1,000 dilution, A-21069 (Thermo Fisher Scientific)).

Next, after washing of the cells with PBST for 10 minutes was performed four times, they were mounted with VECTASHIELD containing DAPI (H-1200 (Vector Laboratories)) and an image was obtained using a Virtual Slide microscope system (VS120 (Olympus)) having a x20 objective lens. The images were analyzed using a neuron J plugin in Image J software and the neurite length tracing and quantification were performed (Meijering, E.; Jacob, M.; Sarria, J. C.; Steiner, P.; Hirling, H.; Unser, M., Design and validation of a tool for neurite tracing and analysis in fluorescence microscopy images. Cytometry A 2004, 58 (2), 167-76.).

### (Statistical method)

Statistical analysis was performed using StatView 5.0 (SAS Institute, Cary, NC, USA). Data were analyzed by one-way ANOVA followed by Tukey-Kramer's test as a post hoc comparison. Significance level was set at p<0.05.

### [Evaluation results of peptide complex]

The peptide complexes thus obtained were subjected to various activity tests using each of the aforesaid methods.

### (Evaluation of affinity for hTrkB)

The binding kinetics (binding affinity K_{D}) of the peptide complexes DiTrbL1 to 3 and DiTrbD1 to 3 to the extracellular domain of the hTrkA-C proteins were measured using the method described above (surface plasmon resonance (SPR): evaluation of binding kinetics (binding affinity K_{D})). Based on the measurements, the binding intensity and selectivity of the peptide complex to hTrkB, compared with that to hTrkA and hTrkC were evaluated.

The SPR response of the peptide complexes (DiTrbL1 to 3 and DiTrbD1 to 3) to hTrkB is shown in Fig. 6 and the binding affinity K_{D} for the hTrkA-C proteins is shown in Table 6.

**[Table 6]**

| **Homodimer** | **TrkA** | **TrkB** | **TrkC** |
|---|---|---|---|
| **DiTrbL1** | N.B. | 60 | N.B. |
| **DiTrbL2** | 7 | 71 | N.B. |
| **DiTrbL3** | N.B. | **2** | N.B. |
| **DiTrbD1** | 29 | 10 | 120 |
| **DiTrbD2** | 4 | 36 | N.B. |
| **DiTrbD3** | 7 | 15 | N.B. |

In the table, the term "N.B." represents non-binding. In other words, it means that no SPR response was detected within a range of the hTrkA-C proteins from 1 to 100 nM.

As expected, the binding affinity K_{D} of the peptide complexes (DiTrbL1 to 3 and DiTrbD1 to 3) for hTrkB was substantially same as that of a monomer form (cyclic peptide), showing that dimerization does not cause a change in the binding ability for hTrkB.

Surprisingly, dimerization caused a significant change in the selectivity to hTrkB. Four of six peptides did not bind to hTrkC or weakly bound thereto (DiTrbD1), while they bound strongly to hTrkA. On the other hand, DiTrbL3, the most powerful hTrkB binder, retained high selectivity to hTrkB and binding to hTrkA and hTrkC was not observed on the SPR sensor chip.

### (Evaluation of phosphorylation inducibility of TrkB)

The agonist activity on TrkB was studied by causing DiTrbL3 showing the highest selectivity for TrkB to induce phosphorylation of mouse TrkB in primary mouse hippocampal neurons. Described specifically, with a natural protein agonist BDNF (0.1 nM, 15 minutes) as a positive control experiment and a DMSO solution as a negative control experiment, whether or not 1 nM DiTrbL3 induced phosphorylation of TrkB was confirmed. More specifically, whether or not phosphorylation of TrkB was induced was confirmed using an anti-phosphorylated Trk antibody by the method described above (western blotting method).

It was clearly detected that exogenous application of BDNF, a natural protein agonist, induced phosphorylation of Trk (Fig. 7(a), Lane 2). On the other hand, the negative DMSO control did not induce phosphorylation of Trk at all (Fig. 7(a), Lane 1). It was found that the exogenous application of DiTrbL3 at 1 nM concentration induced phosphorylation of TrkB at that concentration (Fig. 7(a), Lane 3).

TrkA is not expressed in the hippocampal neurons usually, but the anti-phosphorylated Trk antibody used in the present analysis (western blotting method) recognizes not only TrkB but also TrkA and TrkC, so that it has been confirmed by the following method that DiTrbL3 has specificity to phosphorylation induction of TrkB.

Described specifically, in a manner similar to that described above except for the use of a kinase inhibitor ANA-12 specific to the intracellular catalyst domain of TrkB, a western blotting method using the anti-phosphorylated Trk antibody was performed.

The treatment of the hippocampal neurons with ANA-12 decreased phosphorylation of Trk induced by DiTrbL3 (Fig. 7(b), Lane 3 vs Lane 4). This suggests that DiTrbL3 selectively induces phosphorylation of TrkB. The quantitative analysis of TrkB phosphorylation in the dose titration of DiTrbL3 has revealed that DiTrbL3 obviously induces TrkB phosphorylation even in sub-nanomolar concentration of DiTrbL3 (0.2 nM, p<0.01) (Fig. 7(c)).

Taken together with the in vitro evaluation of the selective binding of DiTrbL3 to hTrkB and the cell assay, DiTrbL3 may be a selective agonist for TrkB phosphorylation and has been found to show cross reactivity with both mouse and human TrkB proteins.

### (Evaluation on activation of signaling kinase downstream of TrkB)

In a manner similar to the aforesaid method (evaluation of phosphorylation inducibility of TrkB), whether or not the signaling kinase downstream of TrkB was activated by the exogenous treatment of DiTrbL3 was studied using the method (western blotting method) described above.

As expected, DiTrbL3 drastically activated phosphorylation of Akt and Erk1/2 in the primary hippocampal neurons (Figs. 8(a) and (b), Lane 3) and in the presence of ANA-12, the phosphorylation activity decreased (Figs. 8(a) and (b), Lane 4). This strongly suggests the dependence of downstream signaling on TrkB.

The quantitative results have shown that with respect to the activation of Akt and Erk1/2, a significant difference is present in induction between DMSO and DiTrbL3 (Figs. 8(c) to (e), p<0.01). Further, they have suggested that phosphorylation of these kinases is suppressed by the treatment with ANA-12 (Figs. 8(c) to (e), p<0.05 for Akt and p<0.01 for Erk1/2) and DiTrbL3 starts the downstream signaling cascade and induces autophosphorylation of TrkB.

The off-target activity of DiTrbL3 by the other receptor tyrosine kinase was measured by the method described above (phospho-RTK array evaluation), that is, an array-based experiment including respectively different 40 RTKs.

Incubation of BDNF and primary hippocampal neurons for one hour caused phosphorylation of TrkB (Fig. 9, bottom left). Very similar to the observation results, DiTrbL3 phosphorylated only TrkB (Fig. 9, top right). Further, the sample treated with ANA-12 showed a reduction in TrkB phosphorylation signal induced by DiTrbL3 in both RTK array and densitometric analysis thereof (Fig. 9, bottom right, and Fig. 10).

It has been proved strongly by the aforesaid results that DiTrbL3 is a specific TrkB agonist.

### (Activation induction of neuronal gene and promotion of axonal outgrowth by DiTrbL3 treatment)

Since BDNF has an important role in the adjustment of synapse plasticity which responds to immediate early genes such as c-fos and Arc, an mRNA expression level of c-fos and Arc under the treatment of DiTrbL3 was quantified.

After the primary cortical neurons were treated for one hour with BDNF (1 nM) or DiTrbL3 (1 nM), RNA extraction and qRT-PCR measurement were performed by the method described above (RNA extraction and qRT-PCR). As a result, the expression of c-fos and Arc significantly increased by the DiTrbL3 treatment (Fig. 11, p<0.01)

Next, the cell level effects of DiTrbL3 treatment on the primary neurons were verified. When the primary neurons were treated with DiTrbL3 for 3 days by the method described above (Immunocytochemistry for evaluation of axonal outgrowth), the axonal length of the primary neurons was elongated as in the BDNF treatment (Fig. 12(a)). The quantitative results have shown that 10 nM and 100 nM DiTrbL3 significantly reinforced the axonal outgrowth (Fig. 12(b), compared with the DMSO treatment, p<0.01).

## Claims

1. A cyclic peptide comprising a structure represented by the following formula (1):
-Xaa¹(Xaa²)ₙ₁-Xaa³-Xaa⁴- (1)
(in the formula (1),
Xaa¹ is a basic amino acid,
Xaa² is an amino acid,
Xaa³ and Xaa⁴ are each independently a basic amino acid or an aromatic amino acid, and
n1 is an integer of 0 to 10), or a pharmacologically acceptable salt of the cyclic peptide.

2. The cyclic peptide or pharmacologically acceptable salt thereof according to Claim 1, wherein the structure represented by the formula (1) is any of structures represented by the following formula (2):
-Arg-(Xaa²)ₙ₁-Phe-Trp- (2)
(in the formula (2),
Xaa² and n1 have the same meanings as defined above).

3. The cyclic peptide or pharmacologically acceptable salt thereof according to Claim 1 or 2, wherein the cyclic structure of the cyclic peptide contains an N-CO-CH₂-S structure.

4. The cyclic peptide or pharmacologically acceptable salt thereof according to Claim 3, wherein the N is derived from the amino group of tyrosine.

5. The cyclic peptide or pharmacologically acceptable salt thereof according to Claim 3 or 4, wherein the S is derived from a thiol group of cysteine.

6. The cyclic peptide or pharmacologically acceptable salt thereof according to any one of Claims 1 to 5, wherein the number of amino acid residues of the cyclic structure of the cyclic peptide is 6 to 20.

7. A cyclic peptide comprising any one of cyclic structures represented by the following formulas (3) to (8): 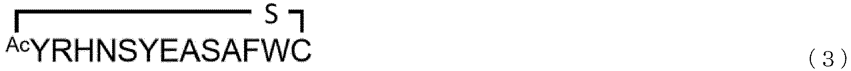 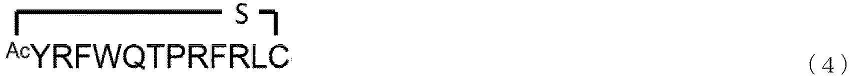 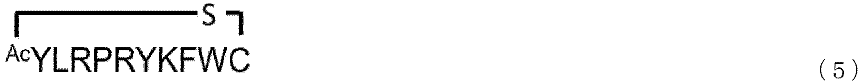 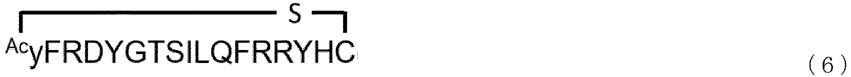 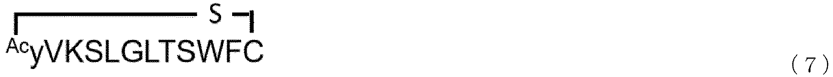 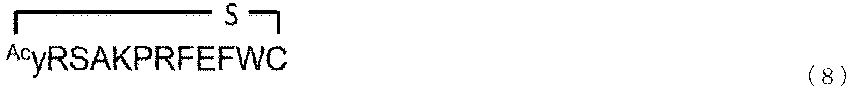 (in the formulas (3) to (8), Ac is -CH₂-CO-, Y is L-tyrosine, and y is D-tyrosine), or a pharmacologically acceptable salt of the cyclic peptide.

8. The cyclic peptide or pharmacologically acceptable salt thereof according to any one of Claims 1 to 7, wherein the cyclic peptide further comprises a linear structure having 1 to 20 amino acids.

9. A peptide complex comprising two cyclic peptides as claimed in any one of Claims 1 to 7, one of the cyclic peptides being linked to the other cyclic peptide via a linker, or a pharmacologically acceptable salt of the peptide complex.

10. The peptide complex or pharmacologically acceptable salt thereof according to Claim 9, wherein the two cyclic peptides are identical.

11. The peptide complex or pharmacologically acceptable salt thereof according to Claim 9 or 10, wherein the linker has a length of 20 Å or more and 200 Å or less.

12. The peptide complex or pharmacologically acceptable salt thereof according to any one of Claims 9 to 11, inducing phosphorylation of TrkB.

13. A pharmaceutical composition comprising at least one selected from the group consisting of the cyclic peptide as claimed in any one of Claims 1 to 7 and the pharmacologically acceptable salt thereof and the peptide complex as claimed in any one of Claims 9 to 12 and the pharmacologically acceptable salt thereof.

14. The pharmaceutical composition according to Claim 13 to be used for the treatment or prevention of neurodegenerative diseases, neurodevelopmental disorders, or neuropsychiatric diseases.
